# EUROPEAN PATENT SPECIFICATION

(11) **EP 3 279 593 B1**
(45) Date of publication and mention of the grant of the patent: **26.02.2020**
(21) Application number: 16771161.3
(22) Date of filing: 18.01.2016
(51) Int. Cl.: F25D 23/12, F25D 17/04

(54) **STEAM-STERILIZATION REFRIGERATOR AND CONTROL METHOD THEREFOR**
DAMPFSTERILISATIONSKÜHLSCHRANK UND STEUERUNGSVERFAHREN DAFÜR
RÉFRIGÉRATEUR DE STÉRILISATION À LA VAPEUR ET SON PROCÉDÉ DE COMMANDE

(30) Priority: 01.04.2015 CN 201510152747; 01.04.2015 CN 201520194293 U; 14.07.2015 CN 201510420188; 14.07.2015 CN 201520513300 U; 14.07.2015 CN 201510416248; 14.07.2015 CN 201520518783 U; 14.07.2015 CN 201510419475; 14.07.2015 CN 201520517933 U; 14.07.2015 CN 201510419445; 14.07.2015 CN 201520513333 U; 14.07.2015 CN 201510416687; 14.07.2015 CN 201520517873 U; 14.07.2015 CN 201510419506; 14.07.2015 CN 201520518525 U; 14.07.2015 CN 201510419924; 14.07.2015 CN 201520517954 U; 14.07.2015 CN 201510419923; 14.07.2015 CN 201520513965 U; 14.07.2015 CN 201510419922; 14.07.2015 CN 201520517936 U; 14.07.2015 CN 201510416250; 14.07.2015 CN 201520518006 U; 14.07.2015 CN 201510420220; 14.07.2015 CN 201520517832 U
(43) Date of publication of application: 07.02.2018
(73) Proprietor: Hefei Hualing Co., Ltd., Hefei, Anhui 230601 (CN); Midea Group Co., Ltd., Foshan, Guangdong 528311 (CN)
(72) Inventor: ZHA, Sheng, Hefei Anhui 230601 (CN); YANG, Song, Hefei Anhui 230601 (CN); ZHANG, Yu, Hefei Anhui 230601 (CN); HU, Peng, Hefei Anhui 230601 (CN); LI, Linfeng, Hefei Anhui 230601 (CN); PAN, Shuwei, Hefei Anhui 230601 (CN); YU, Jian, Hefei Anhui 230601 (CN); HONG, Hu, Hefei Anhui 230601 (CN); LI, Chang, Hefei Anhui 230601 (CN)
(74) Representative: Lam, Alvin
(86) International application number: PCT/CN2016/071244
(87) International publication number: WO 2016/155406

(56) References cited:
- WO-A1-2012/091256
- CN-A- 85 106 716
- CN-A- 102 192 629
- CN-A- 104 792 102
- CN-A- 104 990 328
- CN-A- 104 990 350
- CN-A- 104 990 351
- CN-A- 104 990 352
- CN-A- 104 990 353
- CN-A- 105 091 479
- CN-A- 105 091 480
- CN-A- 105 091 481
- CN-A- 105 091 482
- CN-A- 105 115 233
- CN-A- 105 180 573
- CN-U- 202 361 740
- CN-U- 204 902 408
- CN-U- 204 902 409
- CN-U- 204 902 410
- CN-U- 204 902 411
- CN-U- 204 923 667
- CN-U- 204 923 668
- CN-U- 204 963 392
- CN-U- 204 963 393
- DE-U1- 9 114 724
- JP-A- 2002 233 345
- JP-U- 3 187 441
- KR-A- 20120 011 452
- US-A- 4 138 858

## Description

### FIELD

The present disclosure relates to a field of household appliances, more particularly to a steam sterilization refrigerator and a control method for the steam sterilization refrigerator.

### BACKGROUND

A refrigerating compartment of a refrigerator can store vegetables, fruits, fishes, meats, or other food. After a period of use, the food in the refrigerating compartment of the refrigerator will breed bacteria, and produce unpleasant odor.

In the related art, various solutions are proposed to sterilize and deodorize the refrigerator, for example, the sterilization can be performed by utilizing a filter, ozone, or ultraviolet rays. However, the above sterilization methods have the following defects. First, the ozone and ultraviolet rays are likely to cause harm to a human body; second, the sterilizing effect is poor: the sterilization methods of the related art generally sterilize only the air in the refrigerating compartment, while bacteria on a wall, a rack and a fruit and vegetable box of the refrigerating compartment cannot be killed, and bacteria which are not killed will continue to multiply after the sterilization is completed; third, the structure is complex along with large manufacturing difficulty and high manufacturing cost; fourth, the energy consumption is high because a fan for circulating the air in the refrigerating compartment needs to be used.

WO2012091256 discusses a refrigerator that stores foods at a low point of temperature and removes bacteria of the foods stored in a storage room effectively, to maintain the storage room clean. JP2002233345 discusses a refrigerator capable of disinfecting the inside thereof and preventing germs from invading thereinto from the outside when recovering the pressure from vacuum. JP3187441 discusses a showcase humidifying and cooling device capable of preventing generation of bacteria by a simple constitution.

### SUMMARY

The present disclosure seeks to solve at least one of the problems existing in the related art to at least some extent. To this end, the present disclosure proposes a steam sterilization refrigerator having advantages of a good sterilizing effect, low energy consumption and a simple structure.

According to one aspect, the present invention provides a steam sterilization refrigerator as set out in claim 1.

According to another aspect, the present invention provides a control method for a steam sterilization refrigerator as set out in claim 12.

### BRIEF DESCRIPTION OF THE DRAWINGS

Fig. 1 illustrates a schematic view of a steam sterilization refrigerator according to an embodiment of the present disclosure.
Fig. 2 illustrates a schematic view of a steam sterilization refrigerator according to a first embodiment of the present disclosure.
Fig. 3 illustrates a partially schematic view of the steam sterilization refrigerator according to the first embodiment of the present disclosure.
Fig. 4 illustrates a schematic view of a flexible connecting tube of the steam sterilization refrigerator according to the first embodiment of the present disclosure.
Fig. 5 illustrates a partially schematic view of the steam sterilization refrigerator according to the first embodiment of the present disclosure.
Fig. 6 illustrates a schematic view of a steam sterilization refrigerator according to a second embodiment of the present disclosure.
Fig. 7 illustrates a schematic view of the steam sterilization refrigerator according to the second embodiment of the present disclosure.
Fig. 8 illustrates a schematic view of the steam sterilization refrigerator according to the second embodiment of the present disclosure.
Fig. 9 illustrates a schematic view of a water storage box of the steam sterilization refrigerator according to the second embodiment of the present disclosure.
Fig. 10 illustrates a partially schematic view of the steam sterilization refrigerator according to the second embodiment of the present disclosure.
Fig. 11 illustrates a schematic view of a steam sterilization refrigerator according to a third embodiment of the present disclosure.
Fig. 12 illustrates a partially exploded view of the steam sterilization refrigerator according to the third embodiment of the present disclosure.
Fig. 13 illustrates a sectional view of a water inlet transition member of the steam sterilization refrigerator according to the third embodiment of the present disclosure.
Fig. 14 illustrates a schematic view of a steam sterilization refrigerator according to a fourth embodiment of the present disclosure.
Fig. 15 illustrates a schematic view of a steam sterilization assembly of the steam sterilization refrigerator according to the fourth embodiment of the present disclosure.
Fig. 16 illustrates a schematic view of a steam sterilization device of a steam sterilization refrigerator according to a fifth embodiment of the present disclosure.
Fig. 17 illustrates a schematic view of temperature control and protection for the steam sterilization device of the steam sterilization refrigerator according to the fifth embodiment of the present disclosure.
Fig. 18 illustrates a schematic view of a steam sterilization refrigerator according to a sixth embodiment of the present disclosure.
Fig. 19 illustrates a schematic view of a steam sterilization assembly of the steam sterilization refrigerator according to the sixth embodiment of the present disclosure.
Fig. 20 illustrates a partially schematic view of the steam sterilization refrigerator according to the sixth embodiment of the present disclosure.
Fig. 21 illustrates a partially schematic view of the steam sterilization refrigerator according to the sixth embodiment of the present disclosure.
Fig. 22 illustrates a flow diagram of a control method for a steam sterilization refrigerator according to an embodiment of the present disclosure.
Fig. 23 illustrates a flow diagram of a control method for a steam sterilization refrigerator according to an embodiment of the present disclosure.
Fig. 24 illustrates a flow diagram of a control method for a steam sterilization refrigerator according to an embodiment of the present disclosure.

### DETAILED DESCRIPTION

Embodiments of the present disclosure will be described in detail below, and examples of the embodiments are shown in accompanying drawings. The embodiments described herein with reference to drawings are explanatory, illustrative, and used to generally understand the present disclosure. The embodiments shall not be construed to limit the present disclosure.

A sterilization refrigerator in the related art is provided with an air hose in communication with a refrigerating compartment, and the air hose is provided with a fan therein. When the sterilization refrigerator performs sterilization, the fan operates to suck air in the refrigerating compartment into the air hose. Then the air sucked into the air hose is sterilized to obtain sterile air. Finally, the fan blows the sterile air into the refrigerating compartment. That is to say, under the function of the fan, the air is circulated in the refrigerating compartment and the air hose, and the air is sterilized during the circulation.

However, the sterilization refrigerator has the following defects. First, the sterilizing effect is poor: only the air in the refrigerating compartment can be sterilized by the sterilization refrigerator, but bacteria on a wall of the refrigerating compartment and bacteria attached to accessories (such as a storage box and a rack) of the refrigerating compartment cannot be killed, and the bacteria which are not killed will continue to multiply after the sterilization is completed. Second, the structure is complex along with large manufacturing difficulty and high manufacturing cost: the refrigerator needs to be provided with the air hose in communication with the refrigerating compartment, both an inlet and an outlet of the air hose need to be communicated with the refrigerating compartment, and an sterilization unit and the fan need to be disposed in the air hose, which significantly increases the manufacturing difficulty and manufacturing cost of the refrigerator; additionally, a biosensor and an odor sensor are provided in order to detect a pollution source, which also increases the manufacturing cost of the sterilization refrigerator. Third, the energy consumption is high: during sterilization, the fan is required to operate, which leads to an increase in the energy consumption of the sterilization refrigerator.

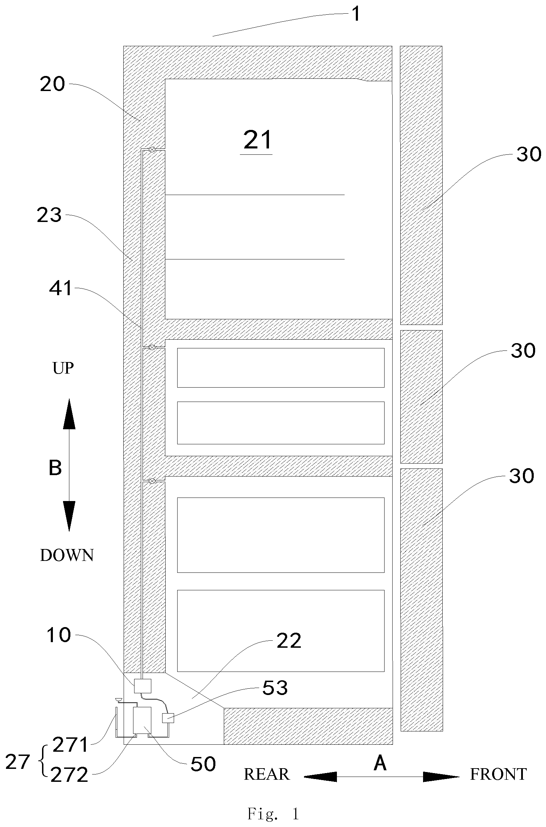

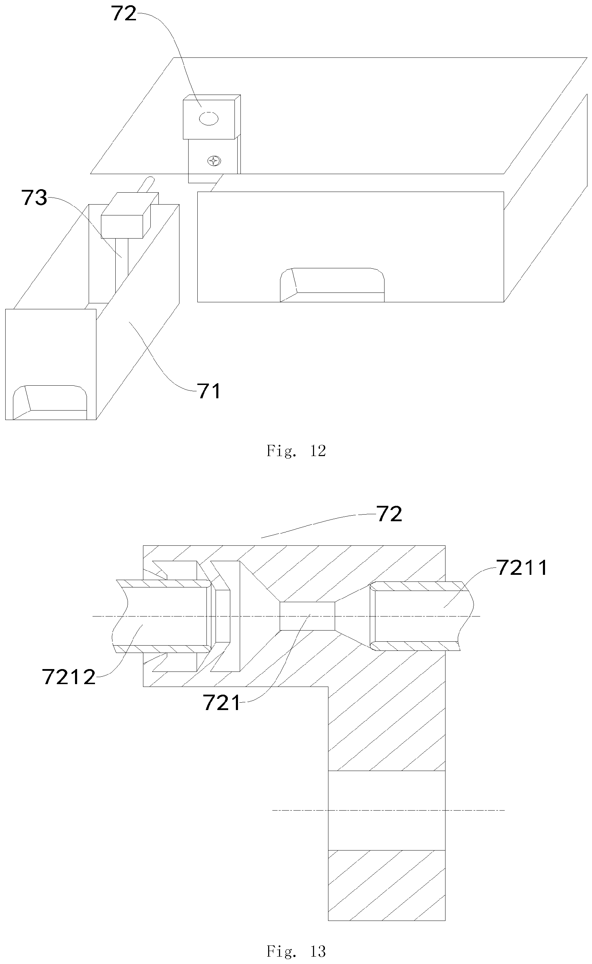

A steam sterilization refrigerator 1 according to an embodiment of the present disclosure will be described below with reference to the drawings. As illustrated in Figs. 1 to 21, the steam sterilization refrigerator 1 according to embodiments of the present disclosure includes a refrigerator body 20, a door body 30 and a steam generator 10.

The refrigerator body 20 has a refrigerating compartment 21 and a compressor chamber 22 therein, and the door body 30 is disposed to the refrigerator body 20. The steam generator 10 is disposed to at least one of the refrigerator body 20 and the door body 30, the steam generator 10 has a steam outlet 1013, and the steam outlet 1013 is in direct communication with the refrigerating compartment 21 or in indirect communication with the refrigerating compartment 21 via a steam conveying channel 40.

In the steam sterilization refrigerator 1 according to embodiments of the present disclosure, by providing the steam generator 10 and communicating the steam outlet 1013 of the steam generator 10 with the refrigerating compartment 21, steam produced by the steam generator 10 can be conveyed into the refrigerating compartment 21. Thus, air in the refrigerating compartment 21 can be sterilized, and also bacteria on a wall of the refrigerating compartment 21 and bacteria attached to accessories (such as a storage box and a rack) in the refrigerating compartment 21 can be killed. Therefore, the steam sterilization refrigerator 1 according to embodiments of the present disclosure has an advantage of a good sterilizing effect.

Moreover, the steam sterilization refrigerator 1 according to embodiments of the present disclosure does not need to evacuate the air in the refrigerating compartment and the steam produced by the steam generator 10 can enter the refrigerating compartment 21 without using a fan. Therefore, the steam sterilization refrigerator 1 according to embodiments of the present disclosure does not need to be provided with an exhaust tube and the fan. The steam sterilization refrigerator 1 according to embodiments of the present disclosure does not need to be provided with the biosensor and the odor sensor, either. Thus, a structure of the steam sterilization refrigerator 1 is simplified, and the manufacturing difficulty and manufacturing cost of the steam sterilization refrigerator 1 is reduced.

In addition, when the steam sterilization refrigerator 1 performs sterilization, since the steam needs to be supplied into the refrigerating compartment 21, articles in the refrigerating compartment 21 are required to be taken out before the sterilization. Thus, during the sterilization, the refrigerating compartment 21 does not need to be refrigerated, and the fan does not need to be operated. Therefore, the steam sterilization refrigerator 1 according to embodiments of the present disclosure further has an advantage of low energy consumption.

Therefore, the steam sterilization refrigerator 1 according to embodiments of the present disclosure has advantages of the good sterilizing effect, the simple structure, the low manufacturing difficulty, the low manufacturing cost, and the low energy consumption.

As illustrated in Figs. 1 to 21, in some embodiments of the present disclosure, the steam sterilization refrigerator 1 includes the refrigerator body 20, the door body 30 and the steam generator 10. The steam outlet 1013 of the steam generator 10 is in direct communication with the refrigerating compartment 21 or in indirect communication with the refrigerating compartment 21 via the steam conveying channel 40.

Specifically, in the present application, the direct communication between the steam outlet 1013 and the refrigerating compartment 1011 means that the steam generator 10 is placed in the refrigerating compartment 21 or the steam generator 10 is disposed to a rear surface of the door body 30, the steam outlet 1013 may not be provided with a valve and/or a segment of tube, and then the steam directly enters the refrigerating compartment 21 after coming out of the steam outlet 1013. Furthermore, the steam outlet 1013 may also be provided with a valve and/or a nozzle and/or a segment of tube (which may also be referred to as a straight-through valve, a straight-through nozzle and a straight-through tube), and then the steam enters the refrigerating compartment 1011 after coming out of the straight-through valve and/or the straight-through nozzle and/or the straight-through tube mounted to the steam outlet 1013. One objective of mounting the straight-through valve and/or the straight-through nozzle and/or the straight-through tube at the steam outlet 1013 is to obtain an additional technical effect. For example, by mounting the straight-through tube at the steam outlet 1013, the steam can be directly conveyed to a preset position; by mounting the straight-through nozzle at the steam outlet 1013, a flow velocity of the steam can be improved.

The indirect communication between the steam outlet 1013 and the refrigerating compartment 21 via the steam conveying channel 40 means that the steam generator 10 is disposed outside the refrigerating compartment 21, a first end of the steam conveying channel 40 is in communication with the steam outlet 1013, and a second end of the steam conveying channel 40 is in communication with the refrigerating compartment 21.

It should be understood by those skilled in the art that, when the refrigerator body 20 has a plurality of refrigerating compartments 21 therein, the steam generator 10 may not be disposed in any one of the refrigerating compartments 21, and then the steam outlet 1013 is in indirect communication with at least one of the refrigerating compartments 21 via the steam conveying channel 40 in communication with the steam outlet 1013. The steam generator 10 may also be disposed in one of the refrigerating compartments 21, and then the steam outlet 1013 is in direct communication with this one of the refrigerating compartments 21 and in indirect communication with the rest of the refrigerating compartments 21 via the steam conveying channel 40. For example, the steam outlet 1013 may be connected to a plurality of tubes, one of the tubes (the straight-through tube) is in direct communication with one refrigerating compartment 21 where the steam generator 10 is placed, and the steam outlet 1013 may be in indirect communication with at least one of the refrigerating compartments 21 without being provided with the steam generator 10, via the rest of the tubes (i.e. the steam conveying channel 40).

As illustrated in Figs. 1, 14 and 15, the steam generator 10 is disposed in the compressor chamber 22, a first side of the steam generator 10 is provided with a inserting part 1014 connected to the refrigerator body 20 in an inserting manner, and a second side of the steam generator 10 opposite to the first side is provided with a connecting part 1015 connected to the refrigerator body 20.

Advantageously, a plurality of steam generators 10 may be provided, such that a sterilization speed of the steam sterilization refrigerator 1 can be improved. The plurality of the steam generators 10 may all be disposed to the refrigerator body 20, or the plurality of the steam generators 10 may all be disposed to the door body 30. The steam sterilization refrigerator 1 further includes a water collecting pipe (not illustrated) configured to collect condensed water in the refrigerating compartment 21, and the water collecting pipe is in communication with a water tank 50.

The steam sterilization refrigerator 1 further includes a plurality of sterilization mode trigger keys, and a temperature sensor configured to measure a temperature in the refrigerating compartment 21. The steam generator 10 operates, according to trigger of the sterilization mode trigger key, in a sterilization mode corresponding to the triggered sterilization mode trigger key. The temperature sensor is connected to the plurality of sterilization mode trigger keys, and the plurality of sterilization mode trigger keys control the steam generator 10 to stop operating when the temperature sensor detects that the temperature in the refrigerating compartment 21 is within a preset temperature range.

That is to say, each of the sterilization mode trigger keys is electrically connected to the steam generator 10 and configured to output a corresponding trigger electrical signal, and the steam generator 10 adjusts a corresponding sterilization mode according to the corresponding trigger electrical signal output by the sterilization mode trigger key.

Specifically, the sterilization mode includes a first sterilization mode, a second sterilization mode and a third sterilization mode. In the first sterilization mode, the steam generator 10 maintains the temperature in the refrigerating compartment 21 within a range of 60 degrees Celsius to 80 degrees Celsius for a first period of time. In the second sterilization mode, the steam generator 10 maintains the temperature in the refrigerating compartment 21 within a range of 80 degrees Celsius to 95 degrees Celsius for a second period of time. In the third sterilization mode, the steam generator 10 maintains the temperature in the refrigerating compartment 21 within a range of 40 degrees Celsius to 60 degrees Celsius for a third period of time.

That is to say, in the third sterilization mode, the temperature in the refrigerating compartment 21 is greater than or equal to 40 degrees Celsius and smaller than 60 degrees Celsius; in the second sterilization mode, the temperature in the refrigerating compartment 21 is greater than or equal to 60 degrees Celsius and smaller than 80 degrees Celsius; in the first sterilization mode, the temperature in the refrigerating compartment 21 is greater than or equal to 80 degrees Celsius and smaller than 95 degrees Celsius.

Advantageously, in the third sterilization mode, the temperature in the refrigerating compartment 21 maintains at 50 degrees Celsius to facilitate sterilization and defrosting; in the second sterilization mode, the temperature in the refrigerating compartment 21 maintains at 70 degrees Celsius to facilitate sterilization; in the first sterilization mode, the temperature in the refrigerating compartment 21 maintains at 90 degrees Celsius to facilitate sterilization and deodorization.

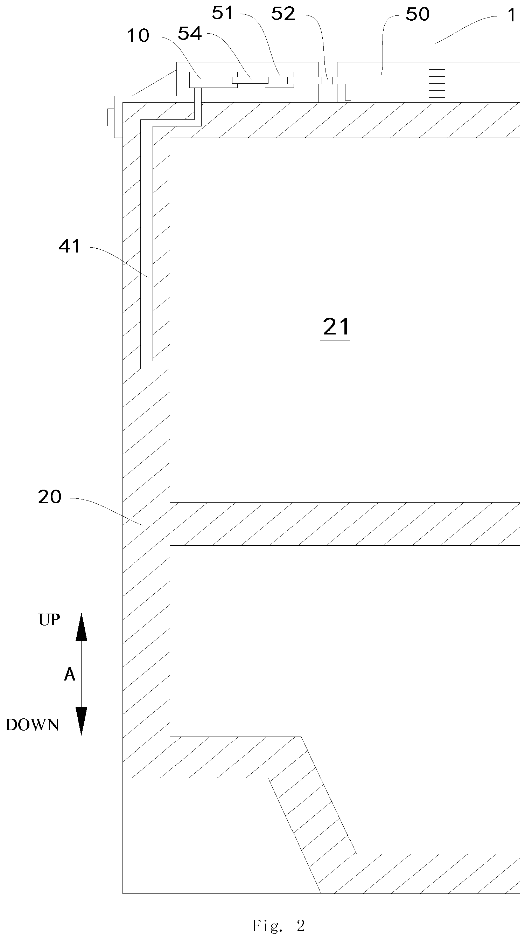

As illustrated Figs. 2 to 4, in a first embodiment of the present disclosure, the steam generator 10 is disposed to one of an upper surface, a left surface, a right surface and a rear surface of the refrigerator body 20, and the steam conveying channel 40 is formed by a steam conveying tube 41. A first end of the steam conveying tube 41 is connected to the steam outlet 1013, and a second end of the steam conveying tube 41 penetrates a foaming layer 23 of the refrigerator body 20 and extends into the refrigerating compartment 21 or is flush with the wall of the refrigerating compartment 21. A front-and-rear direction is as indicated by arrow A in Fig. 1, and an up-and-down direction is as indicated by arrow B in Fig. 2.

By providing the steam generator 10 to one of the upper surface, the left surface, the right surface and the rear surface, the water can be filled in the steam generator 10 conveniently, and also heat dissipation of the steam generator 10 can be facilitated to prevent the heat produced by the steam generator 10 from influencing normal refrigeration of the steam refrigerating compartment 21 when refrigeration is required after completion of the sterilization, thereby avoiding an increase in a load of the steam sterilization refrigerator 1.

As illustrated in Fig. 2, the steam sterilization refrigerator 1 further includes the water tank 50, a flexible connecting tube 51, a first connecting tube 52, a conveying pump 53 and a second connecting tube 54. The water tank 50 is detachably disposed to the upper surface of the refrigerator body 20. A first end of the first connecting tube 52 is connected to the water tank 50, and a second end of the first connecting tube 52 is detachably fitted in a first end of the flexible connecting tube 51. A first end of the second connecting tube 54 is fitted in a second end of the flexible connecting tube 51, and a second end of the second connecting tube 54 is connected to a water inlet of the conveying pump 53. A water outlet of the conveying pump 53 is in communication with a water inlet 1012 of the steam generator 10.

The water in the water tank 50 can be conveyed into the steam generator 10, such that the steam generator 10 can produce the steam continuously. Specifically, the water in the water tank 50 enters the steam generator 10 passing through the first connecting tube 52, the flexible connecting tube 51 and the second connecting tube 54 sequentially.

Since the water tank 50 is detachably disposed to the refrigerator body 20, the water tank 50 can be disassembled for water injection, such that the user can fill water into the water tank 50 conveniently. Furthermore, by detachably fitting the second end of the first connecting tube 52 in the first end of the flexible connecting tube 51, the first connecting tube 52 can be removed along with the water tank 50, and after the water tank 50 is filled with the water, the first connecting tube 52 can be connected to the flexible connecting tube 51 again to facilitate conveying of the water in the water tank 50 into the steam generator 10.

The conveying pump 53 can convey the water in the water tank 50 into the steam generator 10 automatically. Advantageously, the conveying pump 53 is a peristaltic pump, and the second connecting tube 54 is fitted in the peristaltic pump. Thus, the water quantity conveyed into the steam generator 10 can be controlled accurately.

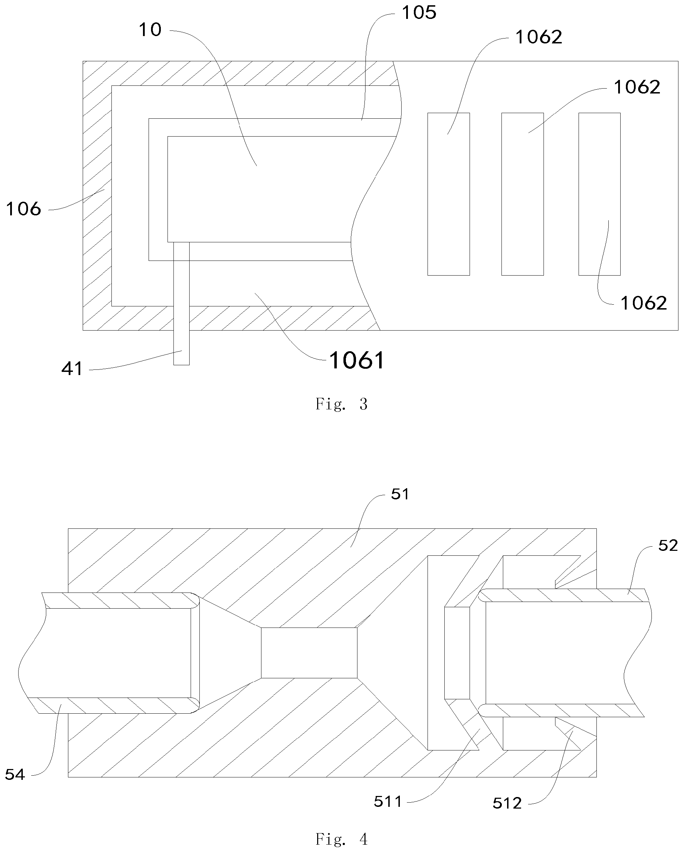

As illustrated in Fig.4, an inner circumferential wall of the first end of the flexible connecting tube 51 is provided with a first annular sealing flange 511 and a second annular sealing flange 512. When the second end of the first connecting tube 52 is fitted in the first end of the flexible connecting tube 51, an end surface of the second end of the first connecting tube 52 abuts against the first annular sealing flange 511, and the second annular sealing flange 512 abuts against a circumferential surface of the first connecting tube 52. Advantageously, the first end of the second connecting tube 54 is in an interference fit with the second end of the flexible connecting tube 51.

As illustrated in Fig. 3, an outer surface of the steam generator 10 may be provided with a thermal insulating layer 105. Thus, the heat produced by the steam generator 10 is prevented from transferring outwardly, such that heating efficiency of the steam generator 10 can be improved, and also the steam generator 10 with high temperature can be prevented from being exposed. On the one hand, the user is prevented from being scalded; on the other hand, the heat radiated by the steam generator 10 can be further prevented from increasing the energy consumption of the steam sterilization refrigerator 1. Specifically, when the steam generator 10 is in operation, a temperature of an outer surface of the thermal insulating layer 105 is not greater than 60 degrees Celsius.

Advantageously, as illustrated in Fig. 3, the steam sterilization refrigerator 1 may further include an outer casing 106, the outer casing 106 may define a cavity 1061 therein, the steam generator 10 and the conveying pump 53 may be disposed in the cavity 1061, and a heat dissipation hole 1062 may be provided in the cavity 1061. Thus, the user can be prevented from being scalded, and also the heat in the outer casing 106 can be exhausted through the heat dissipation hole 1062 conveniently, that is, a part of the heat of the steam generator 10 can radiate to the environment through the heat dissipation hole 1062, such that the temperature of the steam generator 10 can be prevented from being too high.

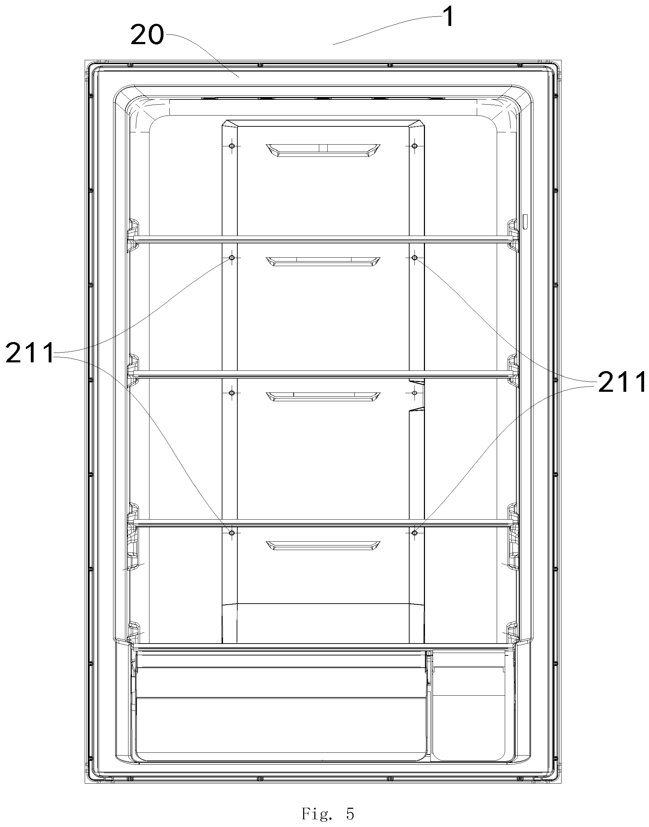

As illustrated in Fig. 5, in an example of the present disclosure, the wall of the refrigerating compartment 21 is provided with a plurality of first steam holes 211 spaced apart from one another in the up-and-down direction. The steam conveying channel 40 is defined in the refrigerator body 20, the steam conveying channel 40 has a plurality of second steam holes in communication with the plurality of first steam holes 211 in one-to-one correspondence. Specifically, the steam conveying channel 40 is defined in the foaming layer of the refrigerator body 20 and in communication with the steam outlet 1013 of the steam generator 10 disposed to the upper surface of the refrigerator body 20.

Thus, the steam produced by the steam generator 10 can be sprayed out through the plurality of first steam holes 211 almost simultaneously, such that the temperature in the refrigerating compartment 21 is rapidly raised to the sterilization temperature, and also the temperature in the refrigerating compartment 21 can be more uniform.

In another example of the present disclosure, the steam conveying channel 40 is formed by the steam conveying tube, a first portion 411 of the steam conveying tube is disposed in the foaming layer 23 of the refrigerator body 20 opposite to the refrigerating compartment 21, and the first portion 411 is provided with the plurality of second steam holes in communication with the plurality of first steam holes 211 in one-to-one correspondence. Specifically, the steam conveying tube is in communication with the steam outlet 1013 of the steam generator 10 disposed to the upper surface of the refrigerator body 20.

Thus, the steam produced by the steam generator 10 can be sprayed out through the plurality of first steam holes 211 almost simultaneously, such that the temperature in the refrigerating compartment 21 is rapidly raised to the sterilization temperature, and also the temperature in the refrigerating compartment 21 can be more uniform.

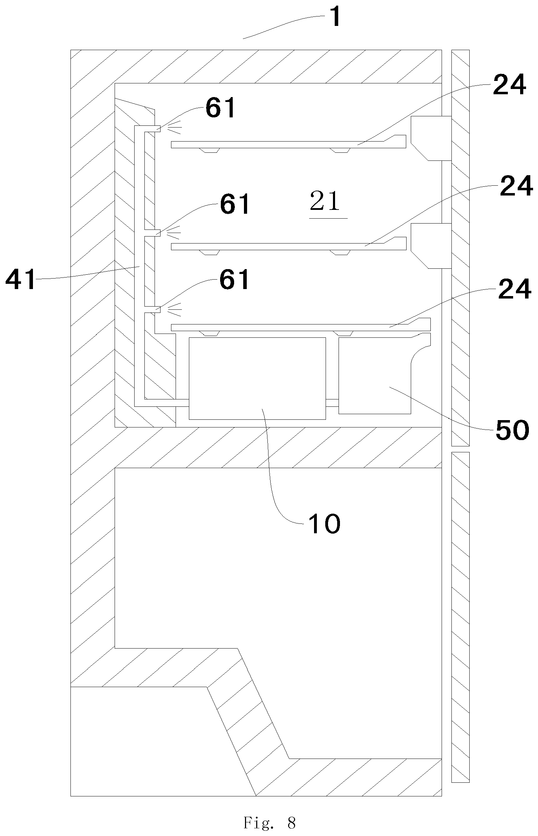

Advantageously, a plurality of racks 24 are disposed in the refrigerating compartment 21 and spaced apart from one another in the up-and-down direction (as illustrated in Fig. 8), each space of a space between adjacent racks 24, a space between a bottom one of the racks 24 and a top wall of the refrigerating compartment 21, and a space between a top one of the racks 24 and the top wall of the refrigerating compartment 21 corresponds to at least one of the first steam holes 211.

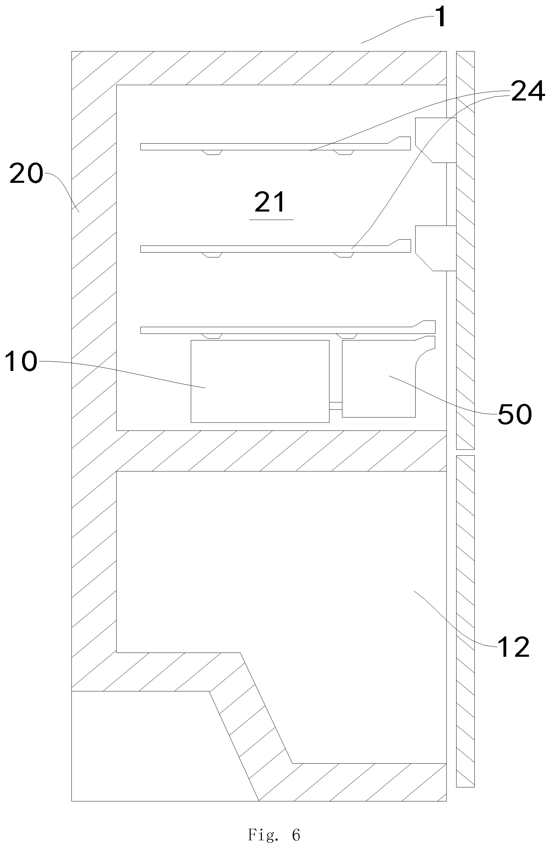

As illustrated in Figs. 6 to 10, in a second embodiment of the present disclosure, the steam generator 10 is disposed in the refrigerating compartment 21, and the steam sterilization refrigerator 1 further includes the water tank 50 disposed in the refrigerating compartment 21. The water tank 50 defines the water outlet, and the water outlet is hermetically connected to the water inlet 1012 of the steam generator 10. The steam outlet 1013 of the steam generator 10 is in direct communication with the refrigerating compartment 21.

By disposing the steam generator 10 in the refrigerating compartment 21, the steam can enter the refrigerating compartment 21 more rapidly, and also the temperature of the steam can be prevented from decreasing during conveying, such that the refrigerating compartment 21 can be sterilized more rapidly and effectively. Furthermore, by disposing the steam generator 10 in the refrigerating compartment 21, the steam conveying channel 40 or other components can be saved, such that the structure of the steam sterilization refrigerator 1 can be simpler.

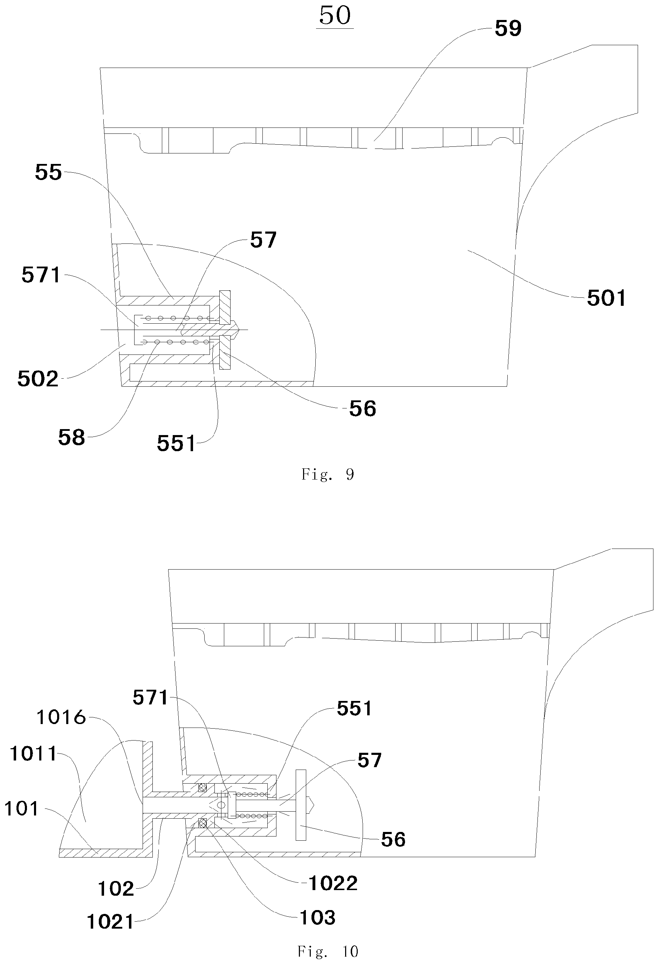

As illustrated in Fig. 10, the steam generator 10 includes a furnace body 101 and a water inlet tube 102, a first through hole 1016 is defined in a wall of the furnace body 101, and the water inlet tube 102 is connected to the first through hole 1016. As illustrated in Figs. 9 and 10, the water tank 50 includes a water tank body 501 and a water outlet tube 55, a second through hole 502 is provided in a wall of the water tank body 501, and the water outlet tube 55 is connected to the second through hole 502. A part of the water inlet tube 102 is fitted in the water outlet tube 55, and a sealing ring 103 is disposed between the part of the water inlet tube and the water outlet tube 55. Thus, the water in the water tank 50 can be prevented from leaking through a gap between the water inlet tube 102 and the water outlet tube 55.

As illustrated in Fig. 10, an outer circumferential surface of the part of the water inlet tube 102 is provided with a first clamping rib 1021 and a second clamping rib 1022 spaced apart from each other. A clamping slot is defined between the first clamping rib 1021 and the second clamping rib 1022, and the sealing ring 103 may be disposed in the clamping slot. Thus, the sealing ring 103 can be mounted more stably, to prevent the sealing ring 103 from dislocation and to ensure a good sealing effect.

The part of the water inlet tube 102 is detachably fitted in the water outlet tube 55. That is to say, the steam generator 10 is detachably mounted to the water tank 50. Thus, when the water is to be added into the water tank 50, the water tank 50 can be detached from the steam generator 10, such that the water tank 50 can be taken out of the refrigerating compartment 21 and the water can be added into the water tank 50 more conveniently. After the water injection is completed, the water tank 50 can be placed back into the refrigerating compartment 21, and the water tank 50 can be assembled with the steam generator 10 again.

As illustrated in Figs. 9 and 10, the water tank 50 further includes a valve disc 56, a valve stem 57 and an elastic element 58. The valve disc 56 is movable between a closed position where an inlet of the water outlet tube 55 is closed and an open position where the inlet of the water outlet tube 55 is open. In other words, when the valve disc 56 is in the closed position, the valve disc 56 can close the water outlet tube 55, and when the valve disc 56 is in the open position, the valve disc 56 can be separated from the water outlet tube 55 to open the water outlet tube 55. The valve stem 57 is disposed in the water outlet tube 55, and the valve stem 57 is connected to the valve disc 56.

When the water inlet tube 102 is inserted into and fitted in the water outlet tube 55, the valve stem 57 is driven to move the valve disc 56 from the closed position to the open position, such that the water outlet tube 55 is communicated with the water inlet tube 102 to supply the water in the water tank 50 into the steam generator 10. The elastic element 58 is disposed between the valve stem 57 and the water outlet tube 55 and normally pushes the valve stem 57 and the valve disc 56 towards the closed position.

Thus, when the water inlet tube 102 is not inserted into the water outlet tube 55, since the elastic element 58 normally pushes the valve stem 57 and the valve disc 56 towards the closed position, the valve disc 56 is in the closed position, and the water outlet tube 55 is closed by the valve disc 56, such that the water in the water tank 50 can be prevent from leaking from the water outlet tube 55.

While the water inlet tube 102 is inserted into the water outlet tube 55, the water inlet tube 102 can drive the valve stem 57 to move, and further the valve stem 57 can drive the valve disc 56 to move towards the open position to open the water outlet tube 55, such that the water in the water tank 50 can be supplied into the steam generator 10 through the water outlet tube 55 and the water inlet tube 102.

As illustrated in Figs. 9 and 10, an end of the valve stem 57 away from the valve disc 56 may be provided with a baffle 571, and an inner wall of the water outlet tube 55 may be provided with an annular rib 551. The elastic element 58 is fitted over the valve stem 57, and located between the baffle 571 and the rib 551. Thus, the elastic element 58 can be mounted more stably. Specifically, the elastic element 58 abuts against the baffle 571 and the rib 551. The elastic element 58 maybe a spring.

As illustrated in Fig. 10, the water inlet tube 102 may be disposed outside the furnace body 101, and an end of the water inlet tube 102 is fixed to an wall of the furnace body 101; the water outlet tube 55 may be disposed in the water tank 50, and an end of the water outlet tube 55 is opened and fixed to a wall of the water tank 50. Thus, the steam generator 10 and the water tank 50 can have a more reasonable structure.

Advantageously, the wall of the refrigerating compartment 21 is provided with a first limiting member, the water tank 50 is provided with a second limiting member 59 fitted with the first limiting member, and when the second limiting member 59 is in contact with the first limiting member, the water tank 50 is spaced apart from the steam generator 10 at a preset distance. By spacing the water tank 50 from the steam generator 10 at the preset distance, the water tank 50 can be prevented from being too close to the steam generator 10, so as to prevent the high temperature of the steam generator 10 from damaging the water tank 50.

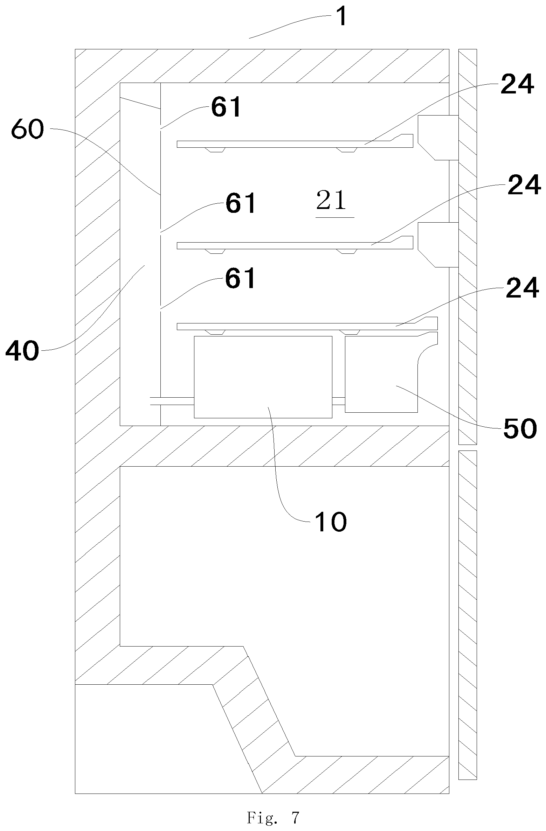

As illustrated in Fig. 7, the steam sterilization refrigerator 1 further includes the plurality of racks 24 and an air duct cover plate 60. The plurality of racks 24 are disposed in the refrigerating compartment 21 and spaced apart from one another in the up-and-down direction, and the steam generator 10 and the water tank 50 are disposed between the bottom wall of the refrigerating compartment 21 and the bottom one of the racks 21. The air duct cover plate 60 is disposed in the refrigerating compartment 21 and located behind the plurality of racks 24, and the steam conveying channel 40 is defined between the air duct cover plate 60 and the refrigerating compartment 21. In other words, as illustrated in Fig. 7, a gas channel is defined between the air duct cover plate 60 and the wall of the refrigerating compartment 21, and the gas channel may be communicated with the steam outlet 1013.

A plurality of steam holes 61 in communication with the steam conveying channel 40 are provided in the air duct cover plate 60 and spaced apart from one another in the up-and-down direction, and each space of the space between adjacent racks 24 and the space between the top one of the racks 24 and the top wall of the refrigerating compartment 21 corresponds to at least one of the steam holes 61. That is to say, the space between adjacent racks corresponds to at least one of the steam holes 61, and the space between the top one of the racks 24 and the top wall of the refrigerating compartment 21 corresponds to at least one of the steam holes 61.

Thus, the steam produced by the steam generator 10 can be sprayed out through the plurality of steam holes 61 almost simultaneously, such that the temperature in the refrigerating compartment 21 is rapidly raised to the sterilization temperature, and also the temperature in the refrigerating compartment 21 can be more uniform, that is, a temperature difference between the space between adjacent racks 24 and the space between the top one of the racks 24 and the top wall of the refrigerating compartment 21 can be minimized.

As illustrated in Fig. 8, the steam sterilization refrigerator 1 further includes the plurality of racks 24, the plurality of racks 24 are disposed in the refrigerating compartment 21 and spaced apart from one another in the up-and-down direction, and the steam generator 10 and the water tank 50 are disposed between the bottom wall of the refrigerating compartment 21 and the bottom one of the racks 21. The steam conveying channel 40 is formed by the steam conveying tube 41, the steam conveying tube 41 is disposed in the refrigerating compartment 21, a lower port of the steam conveying tube 41 is in communication with the steam outlet 1013, and the plurality of steam holes 61 are provided in the steam conveying tube 41 and spaced apart from one another in the up-and-down direction.

Each space of the space between adjacent racks 24 and the space between the top one of the racks 24 and the top wall of the refrigerating compartment 21 corresponds to at least one of the steam holes 61. That is to say, the space between adjacent racks corresponds to at least one of the steam holes 61, and the space between the top one of the racks 24 and the top wall of the refrigerating compartment 21 corresponds to at least one of the steam holes 61.

Thus, the steam produced by the steam generator 10 can be sprayed out through the plurality of steam holes 61 almost simultaneously, such that the temperature in the refrigerating compartment 21 is rapidly raised to the sterilization temperature, and also the temperature in the refrigerating compartment 21 can be more uniform, that is, the temperature difference between the space between adjacent racks 24 and the space between the top one of the racks 24 and the top wall of the refrigerating compartment 21 can be minimized.

Specifically, as illustrated in Fig. 8, the steam conveying tube 41 is L-shaped, a horizontal limb of the steam conveying tube 41 is connected to the steam outlet 22, and the plurality of steam holes 61 may be disposed to a vertical limb of the steam conveying tube 41. Areas of respective openings of the plurality of steam holes 61 can be decreased from bottom to top. The temperature difference between the space between adjacent racks 24 and the space between the top one of the racks 24 and the top wall of the refrigerating compartment 21 can be further reduced.

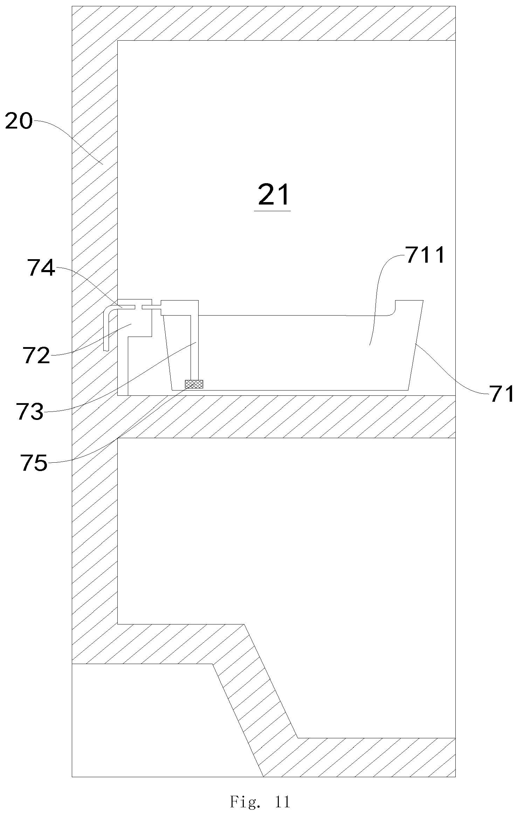

As illustrated in Figs. 11 to 13, in a third embodiment of the present disclosure, the steam sterilization refrigerator 1 further includes a storage box 71 disposed in the refrigerating compartment 21 and capable of being pushed into and pulled out of the refrigerating compartment 21. The storage box 71 defines a storage chamber 711 adapted to accommodate the water and articles, the storage chamber 711 is in communication with the water inlet 1012 of the steam generator 10 to supply the water in the storage chamber 711 into the steam generator 10.

When the steam sterilization refrigerator 1 is in normal operation, the storage box 71 can be used to accommodate articles (such as food), but when the steam sterilization refrigerator 1 needs to be sterilized, the articles in the refrigerating compartment 21 (including the articles in the storage box 71) can be taken out, and the water for producing the steam can be filled into the storage box 71. That is to say, the storage box 71 integrates functions of storing articles and storing the water, and when the steam sterilization refrigerator 1 needs to be sterilized, the storage box 71 serves as the water tank for supplying the water into the steam generator 10.

Since the storage box 71 integrates the functions of storing articles and storing the water, there is no need to provide a separate water tank for supplying the water to the steam generator 10. By communicating the storage chamber 711 of the storage box 71 with the water inlet 1012 of the steam generator 10, there is no need to provide the separate water tank for supplying the water to the steam generator 10, thereby reducing the manufacturing cost of the steam sterilization refrigerator 1.

As illustrated in Figs. 11 to 13, the steam sterilization refrigerator 1 further includes a water inlet transition member 72, a first water inlet tube 73 and a second water inlet tube 74. The water inlet transition member 72 is disposed in the refrigerating compartment 21, the water inlet transition member 72 defines a water flow channel 721 therein, and the water flow channel 721 has an inlet 7211 and an outlet 7212. The first water inlet tube 73 is disposed to the storage box 71, a first end of the first water inlet tube 73 is in communication with the storage chamber 711, and a second end of the first water inlet tube 73 is connected to the inlet 7211 of the water inlet transition member 72 when the storage box 71 is located in the refrigerating compartment 21. A first end of the second water inlet tube 74 is connected to the outlet 7212 of the water inlet transition member 72, and a second end of the second water inlet tube 74 is in communication with the water inlet 1012 of the steam generator 10.

Thus, the water in the storage chamber 711 can be conveyed into the steam generator 10 passing through the first water inlet tube 73, the water inlet transition member 72 and the second water inlet tube 74 sequentially. By disposing the water inlet transition member 72 between the storage box 71 and the steam generator 10, the storage box 71 can be communicated with the steam generator 10 more conveniently and easily, and also the reliability of connection between the storage box 71 and the steam generator 10 can be ensured. When the storage box 71 is pulled out of the refrigerating compartment 21, the storage box 71 is disconnected from the steam generator 10, thereby facilitating the assembly and disassembly thereof.

Specifically, the water inlet transition member 72 may be disposed to a rear wall surface of the refrigerating compartment 21, the first end of the first water inlet tube 31 extends into a bottom of the storage chamber 711, the second end of the first water inlet tube 73 extends into the water flow channel 721 via the inlet 7211, and the first end of the second water inlet tube 74 extends into the water flow channel 721 via the outlet 7212. When the storage box 71 is pulled out of the refrigerating compartment 21, the second end of the first water inlet tube 73 is separated from the water flow channel 721, and the storage box 71 is no longer communicated with the steam generator 10. When the storage box 71 is pushed into the refrigerating compartment 21, the second end of the first water inlet tube 73 extends into the water flow channel 721 via the inlet 7211 again, and in this case the storage box 71 is communicated with the steam generator 10.

As illustrated in Fig. 11, advantageously, the first end of the first water inlet tube 73 is provided with a filter 75. Thus, impurities in the water of the storage box 71 can be prevented from entering the water inlet transition member 72 and the steam generator 10, so as to avoid blockage. Moreover, by disposing the filter 75 to the first end of the first water inlet tube 73, cleanness of the water inlet transition member 72 and the steam generator 10 can be ensured, and cleaning difficulty and frequency can be reduced, thereby facilitating extension of a service life of the steam generator 10.

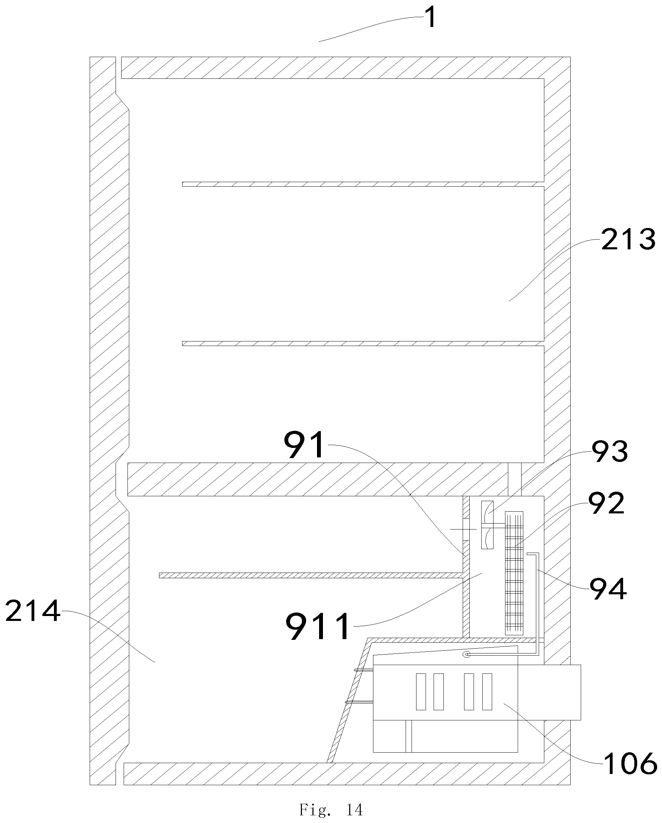

As illustrated in Figs. 14 to 15, in a fourth embodiment of the present disclosure, the steam sterilization refrigerator 1 further includes an air duct component 91, an evaporator 92 and a fan 93. A ventilating channel 911 is defined in the air duct component 91, or the ventilating channel 911 is defined between the air duct component 91 and the wall of the refrigerating compartment 21. The ventilating channel 911 is in communication with each of the refrigerating compartment 21 and the steam outlet 1013, and the steam conveying channel 40 is formed by the ventilating channel 911. The evaporator 92 is disposed in the ventilating channel 911, and the fan 93 is disposed in the ventilating channel 911 to convey the steam and cold air into the refrigerating compartment 21.

That is to say, the steam sterilization refrigerator 1 is an air-cooled refrigerator, and the air duct component 91 constitutes an air channel of the air-cooled refrigerator. In other words, the air-cooled refrigerator conveys the steam into the refrigerating compartment 21 by means of the air channel. Specifically, when the steam sterilization refrigerator 1 refrigerates, the fan 93 is configured to convey the cold air cooled by the evaporator 92 into the refrigerating compartment 21; when the steam sterilization refrigerator 1 is sterilized, the fan 93 is configured to convey the steam produced by the steam generator 10 into the refrigerating compartment 21. In other words, the steam sterilization refrigerator 1 does not need to be provided with an additional fan.

When the known sterilization refrigerator is the air-cooled refrigerator, it needs to be provided with the additional fan to suck the air in the refrigerating compartment into the air hose, and to blow the sterile air in the air hose into the refrigerating compartment. That is to say, when the known sterilization refrigerator is the air-cooled refrigerator, it needs to be provided with at least two fans, one fan is configured to convey the cold air into the refrigerating compartment, and the other fan is configured to suck the air in the refrigerating compartment into the air hose and to blow the sterile air in the air hose into the refrigerating compartment.

The steam sterilization refrigerator 1 according to embodiments of the present disclosure conveys the steam by utilizing the air channel existing in the air-cooled refrigerator, such that there is no need to additionally provide any component configured to convey the steam, and there is no need to greatly reconstruct a production line of the existing air-cooled refrigerator. Moreover, since the high-temperature steam produced by the steam generator 10 enters the ventilating channel 911 and the evaporator 92 is located in the ventilating channel 911, the evaporator 92 can be defrosted by utilizing the high-temperature steam. That is to say, while the steam sterilization refrigerator 1 is being sterilized, the evaporator 92 can also be defrosted.

Advantageously, as illustrated in Fig. 14, the steam generator 10 may be disposed in the compressor chamber 22 to facilitate a purpose of achieving rapid heat dissipation.

As illustrated in Fig. 14, the refrigerator body 20 has a refrigerating chamber 213 and a freezing chamber 214 therein, the ventilating channel 911 is in communication with each of the refrigerating chamber 213, the freezing chamber 214 and the steam outlet 1013. Specifically, the ventilating channel 911 includes a freezing air duct disposed in a freezing liner, a refrigerating air duct configured to convey the cold air and the steam to the refrigerating chamber 213, and an air return channel configured to return the cold air in the refrigerating chamber 213 into the freezing chamber 214. In other words, the freezing air duct is disposed in the freezing chamber 214, i.e. the freezing chamber 214 includes a mounting space configured to place the freezing air duct and a freezing space configured to store articles.

As illustrated in Fig. 14, the steam sterilization refrigerator 1 further includes a steam connecting tube 94, a first end of the steam connecting tube 94 is connected to the steam outlet 1013, a second end of the steam connecting tube 94 extends into the ventilating channel 911, and an opening of the second end of the steam connecting tube 94 faces towards the evaporator 92. Thus, the high-temperature steam sprayed out of the opening of the second end of the steam connecting tube 94 can be directly sprayed to the evaporator 92, so as to prevent the high-temperature steam from directly spraying to components of the steam sterilization refrigerator 1 made of nonmetallic materials.

Since the evaporator 92 of the steam sterilization refrigerator 1 is a metal member, the evaporator 92 can bear a certain high-temperature impact. By making the opening of the second end of the steam connecting tube 94 face the evaporator 92, the high-temperature steam sprayed out of the opening of the second end of the steam connecting tube 94 can be prevented from damaging the components of the steam sterilization refrigerator 1 made of nonmetallic materials. Thus, the service life and the reliability of the steam sterilization refrigerator 1 can be prevented from being influenced, a defrosting effect can be further improved, and a refrigerating effect of the steam sterilization refrigerator 1 can be ensured.

Advantageously, the second end of the steam connecting tube 94 is adjacent to the evaporator 92. More advantageously, the second end of the steam connecting tube 94 is opposite to the evaporator 92.

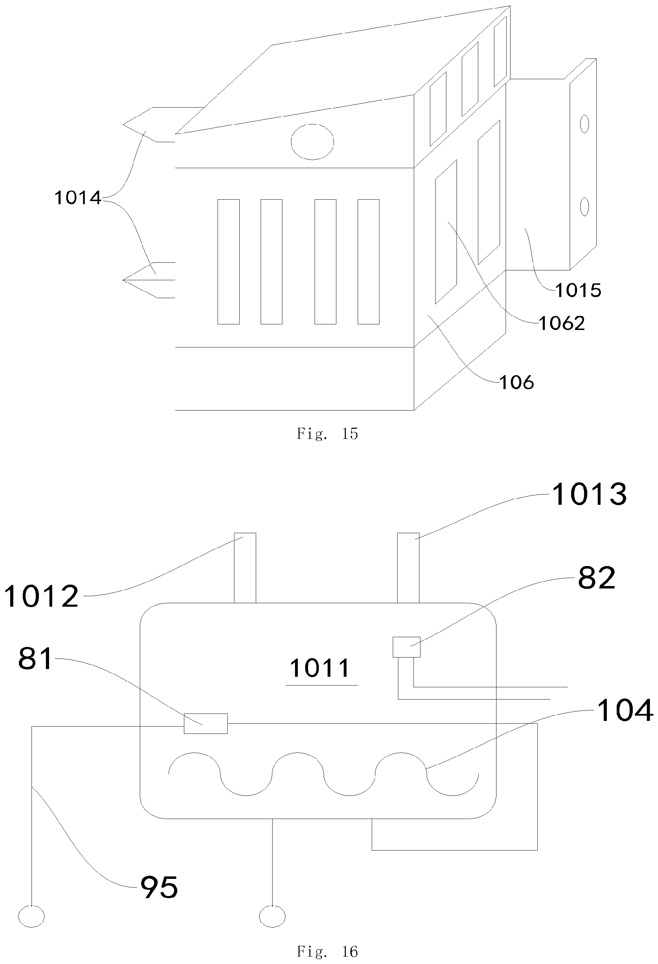

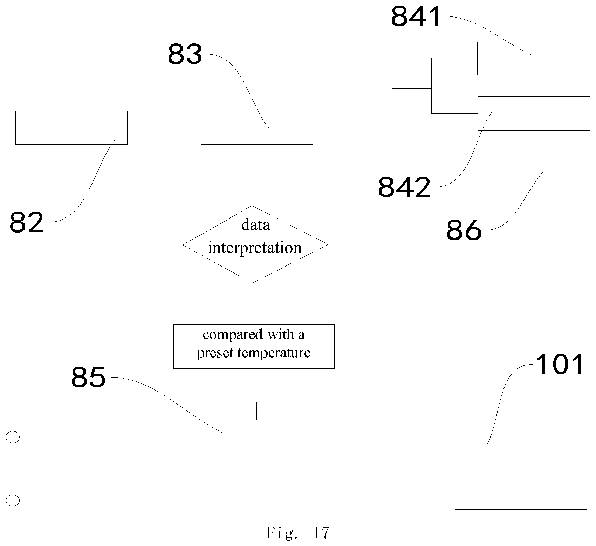

As illustrated in Figs. 16 and 17, in a fifth embodiment of the present disclosure, the steam generator 10 includes the furnace body 101 and an electric heater 104, the furnace body 101 defines the accommodating cavity 1011 configured to accommodate the water, and the accommodating cavity 1011 defines the water inlet 1012 and the steam outlet 1013. The electric heater 104 is adapted to be connected to a power source through a power cord 95 to produce the heat.

The steam sterilization refrigerator 1 further includes the conveying pump 53, a temperature control protector 81, a temperature sensor 82 and a controller 83. The water outlet of the conveying pump 53 is in communication with the water inlet 1012 of the steam generator 10. The temperature control protector 81 is disposed to an outer surface of the furnace body 101 and configured to sense a temperature of the outer surface of the furnace body 101. The temperature control protector 81 is connected to the power cord 95 and automatically disconnected from the power cord 95 when the temperature of the outer surface of the furnace body 101 is greater than or equal to a preset temperature. The temperature sensor 82 is disposed to the outer surface of the furnace body 101 and configured to sense the temperature of the outer surface of the furnace body 101. The controller 83 is connected to the temperature sensor 82 and the electric heater 104, and controls the electric heater 104 to stop heating when the temperature of the outer surface of the furnace body 101 is greater than or equal to the preset temperature.

During the operation of the steam sterilization refrigerator 1, the temperature of the furnace body 101 is maintained within a stable temperature value range according to the sterilization temperature, the power of the electric heater 104, the water flow rate, or other factors. In the case where the sterilization temperature and the power of the electric heater 104 are definite, variation of the water flow rate will lead to an abnormal increase in the temperature of the furnace body 101. The temperature control protector 81 is configured to sense the temperature of the outer surface of the furnace body 101, and the temperature sensor 82 is configured to measure the temperature of the outer surface of the furnace body 101.

Specifically, when the temperature of the outer surface of the furnace body 101 is smaller than the preset temperature, the temperature control protector 81 is turned on to communicate the electric heater 104 and the power source, and in this case the steam sterilization refrigerator 1 operates normally. When the temperature of the outer surface of the furnace body 101 is greater than or equal to the preset temperature, the temperature control protector 81 is turned off automatically to disconnect the electric heater 104 from the power source, and in this case the steam sterilization refrigerator 1 stops operating.

The temperature sensor 82 measures the temperature of the outer surface of the furnace body 101 in real time, and the measured temperature value of the temperature sensor 82 is fed back to the controller 83 in real time. Specifically, when the measured temperature value of the temperature sensor 82 is smaller than the preset temperature (i.e. when the temperature of the outer surface of the furnace body 101 is smaller than the preset temperature), the controller 83 controls the electric heater 104 to communicate with the power source, such that the steam generator 10 can operate normally. When the measured temperature value of the temperature sensor 82 is greater than or equal to the preset temperature (i.e. when the temperature of the outer surface of the furnace body 101 is greater than or equal to the preset temperature), the controller 83 controls the electric heater 104 to disconnect from the power source, such that the steam generator 104 can stop heating.

In the steam sterilization refrigerator 1 according to embodiments of the present disclosure, by disposing the temperature control protector 81 and temperature sensor 82 to the outer surface of the furnace body 101, a dual dry heating-resistant protection can be formed, such that even if one of the temperature control protector 81 and the temperature sensor 82 malfunctions, the other of the temperature control protector 81 and the temperature sensor 82 still can be used for protection. Thus, the safety performance of the steam generator 10 can be significantly improved, and further the safety performance of the steam sterilization refrigerator 1 can be improved.

Specifically, the temperature control protector 102 is a mechanical temperature controller, such as a temperature control tripping protector. When the temperature of the outer surface of the furnace body 101 is greater than or equal to the preset temperature, the temperature control tripping protector trips automatically to disconnect the electric heater 104 from the power source, thereby cutting off the power supply to the electric heater 104.

As illustrated in Fig. 17, the steam sterilization refrigerator 1 further includes a relay 85, the relay 85 is connected to the power cord 95, and the controller 83 is connected to the relay 85 to control the communication and disconnection of the electric heater 104 and the power source through the relay 85. Thus, the controller 83 can control the communication and disconnection of the electric heater 104 and the power source through the relay 85.

Specifically, when the measured temperature value of the temperature sensor 82 is smaller than the preset temperature, the controller 83 control the relay 85 to turn on to communicate the electric heater 104 and the power source, in which case the electric heater 104 operates normally. When the measured temperature value of the temperature sensor 82 is greater than or equal to the preset temperature, the controller 83 control the relay 85 to turn off to disconnect the electric heater 104 from the power source, in which case the electric heater 104 stops heating.

Advantageously, the controller 83 is connected to an electric motor of the conveying pump 53 so as to monitor a working current of the electric motor of the conveying pump 53. The controller 83 can monitor the working current of the electric motor of the conveying pump 53 by means of a known method for monitoring a current. By utilizing the controller 83 to monitor the working current of the electric motor of the conveying pump 53, a failure mode that possibly occurs, such as conveying pump abnormality, a water shortage in the water tank, pipeline clogging, and so on, can be prejudged depending on the working current of the electric motor of the conveying pump 53.

Specifically, when the working current of the electric motor of the conveying pump 53 is within the preset range, and the temperature of the outer surface of the furnace body 101 increases abnormally (the electric heater 104 is disconnected from the power source), this means that the water of the water tank 50 is insufficient or the conveying pump 53 is abnormal (that is, the water output by the conveying pump 53 does not flow into the water tank 50). When the working current of the electric motor of the conveying pump 53 exceeds the preset range, and the temperature of the outer surface of the furnace body 101 increases abnormally (the electric heater 104 is disconnected from the power source), this means that the pipeline is clogged.

The preset range (normal range) may be a range formed by a no-load current and a full-load current of the electric motor of the conveying pump 53. In other words, the working current of the electric motor of the conveying pump 53 is greater than or equal to the no-load current of the electric motor of the conveying pump 53 and smaller than or equal to the full-load current of the electric motor of the conveying pump 53, the working current of the electric motor of the conveying pump 53 being within the preset range.

The steam sterilization refrigerator 1 further includes a first indicator light 841 and a second indicator light 842, and the each of the first indicator light 841 and the second indicator light 842 is connected to the controller 83. When the working current of the electric motor of the conveying pump 53 is within the preset range and the electric heater 104 stops heating (the temperature of the outer surface of the furnace body 101 increases abnormally, and the electric heater 104 is disconnected from the power source), the controller 83 controls the first indicator light 841 to glow or flicker; when the working current of the electric motor of the conveying pump 53 exceeds the preset range and the electric heater stops heating (the temperature of the outer surface of the furnace body 101 increases abnormally, and the electric heater 104 is disconnected from the power source), the controller 83 controls the second indicator light 842 to glow or flicker. Thus, the user can determine the failure reason of the steam generator 10 by observing the first indicator light 841 and/or the second indicator light 842.

In another example of the present disclosure, the steam sterilization refrigerator 1 further includes a voice reminder 86, and the voice reminder 86 is connected to the controller 83. When the working current of the electric motor of the conveying pump 53 is within the preset range and the electric heater 104 stops heating, the controller 83 controls the voice reminder 86 to give out a first reminding voice; when the working current of the electric motor of the conveying pump 53 exceeds the preset range and the electric heater stops heating, the controller 83 controls the voice reminder 86 to give out a second reminding voice. Thus, the user can determine the failure reason of the steam generator 10 by listening to the first reminding voice and/or the second reminding voice given out by the voice reminder 86.

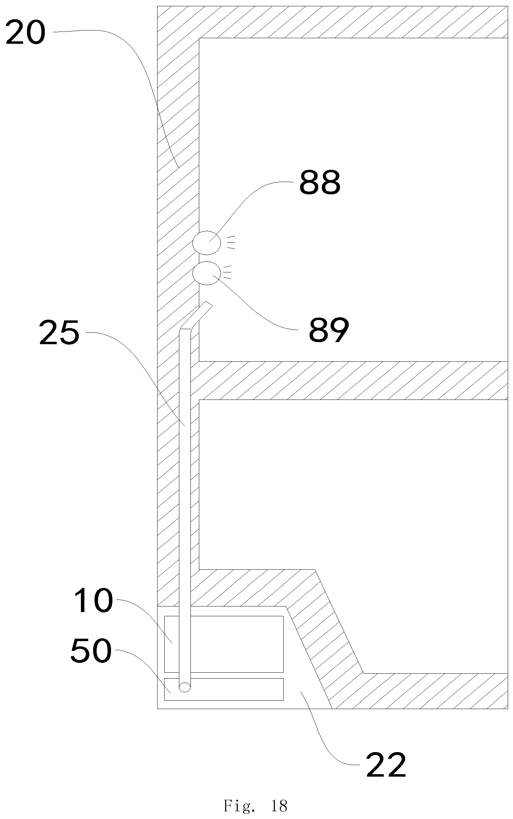

As illustrated in Figs. 18 to 21, in a sixth embodiment of the present disclosure, the steam sterilization refrigerator 1 further includes a water level gauge 87 configured to detect a water level in the water tank 50, a water-shortage indicator light 88, a full-water indicator light 89 and the controller 83. The water-shortage indicator light 88 and the full-water indicator light 89 are disposed in the refrigerating compartment 21. The controller 83 is connected to each of the water level gauge 87, the water-shortage indicator light 88, the full-water indicator light 89 and the steam generator 10 separately. When a water level detection value of the water level gauge 87 is smaller than a first preset value, the controller 83 controls the water-shortage indicator light 88 to glow or flicker, and when the water level detection value is greater than a second preset value, the controller 83 controls the full-water indicator light 89 to light or flicker. The first preset value is smaller than the second preset value.

The water level of the water tank 50 can be detected by the water level gauge 87 before the steam sterilization refrigerator 1 is sterilized. If the full-water indicator light 89 does not glow or flicker, this indicates that the water tank 50 is not full of the water, and the water can be added into the water tank 50 until the full-water indicator light 89 glows or flickers. Thus, the steam generator 10 can be restarted after the water tank 50 is full of the water, such that a dry heating phenomenon can be prevented during the operation of the steam generator 10.

Moreover, when the steam generator 10 continuously produces the steam, the water in the water tank 50 is continuously supplied into the steam generator 10, that is, the water level of the water tank 50 descends continuously. When the water level gauge 87 detects that the water level of the water tank 50 is lower than the first preset value, the controller 83 controls the steam generator 10 to stop operating, and controls the water-shortage indicator light 88 to glow or flicker, to avoid the dry heating of the steam generator 10, such that the safety of the steam generator 10 can be improved. After opening the door body 30 of the steam sterilization refrigerator 1, the user will see that the water-shortage indicator light 88 is glowing or flickering, such that the user may understand that the reason why the steam generator 10 stops operating is that the water level of the water tank 50 is lower than the first preset value.

Specifically, the water level gauge 87 may be a magnetic water level gauge, and the magnetic water level gauge can measure the water level of the water tank 50 accurately, and the magnetic water level gauge is sensitive.

As illustrated in Fig. 1, the steam sterilization refrigerator 1 further includes a water level indicating mechanism 27 configured to indicate the water level of the water tank 50. Specifically, an opening is provided in a side wall of the water tank 50, a lower edge of the opening is flush with an inner bottom surface of the water tank 50, a water level indicating mechanism 27 includes an indicating tube, and a lower end of the indicating tube is connected to the opening. In addition, the water level indicating mechanism 27 includes an indicating slot provided in the side wall of the water tank 50 and a transparent member hermetically covering the indicating slot.

In an embodiment of the present disclosure, the water level indicating mechanism 27 includes a U-shaped tube 271 and an opening 272 provided in the bottom of the water tank 50, an end of the U-shaped tube 271 is in communication with the opening 272 of the water tank 50, and the other end of the U-shaped tube 271 is higher than a top end of the water tank 50 or is flush with the top end of the water tank 50.

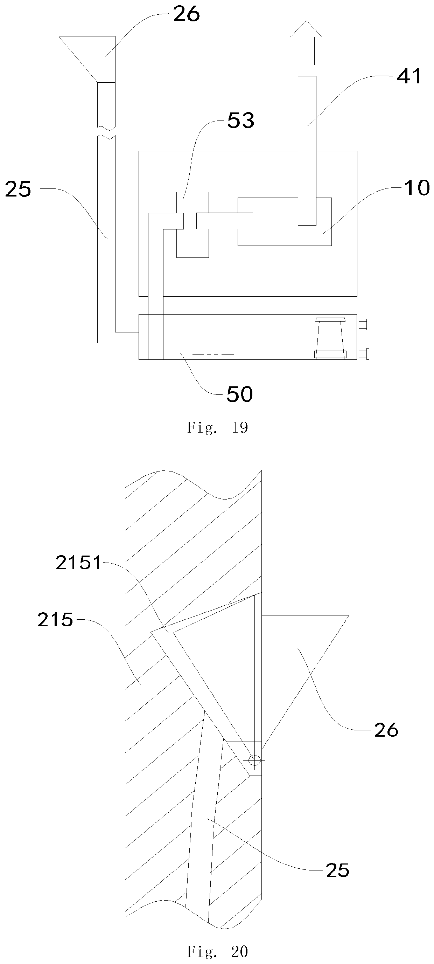

As illustrated in Figs. 19 and 20, the steam sterilization refrigerator 1 may include a water feed tube 25 and a water feed funnel 26, a lower end of the water feed tube 25 is in communication with the water tank 50, and the water feed funnel 26 is disposed to an upper end of the water feed tube 25. When the water is fed into the water tank 50, the water can be fed into the water feed funnel 26, and then the water enters the water tank 50 through the water feed tube 25. By providing the water feed tube 25 in communication with the water tank 50 and disposing the water feed funnel 26 to the water feed tube 25, the water can be fed into the water tank 50 conveniently and easily.

As illustrated in Fig. 20, an upper part of the water feed tube 25 is located in the foaming layer of the refrigerator body 20, the water feed funnel 26 is disposed to the upper end of the water feed tube 25 and is pivotable between an initial position and a water feed position. In the initial position, an outer wall surface of the water feed funnel 26 is flush with a wall 215 of the refrigerating compartment 21, and in the water feed position, at least a part of the water feed funnel 26 protrudes from the wall 215 of the refrigerating compartment 21. When the water needs to be fed into the water tank 50, the water feed funnel 26 can be pivoted from the initial position to the water feed position. When the water feed is completed, the water feed funnel 26 can be pivoted from the water feed position to the initial position. Thus, the space of the refrigerating compartment 21 can be saved, that is, a storage space of the refrigerating compartment 21 can be increased, and also the steam sterilization refrigerator 1 can be more artistic.

Advantageously, as illustrated in Fig. 20, a groove 2151 is provided in the wall 215 of the refrigerating compartment 21, and in the initial position, the water feed funnel 26 is accommodated in the groove 2151. Thus, the steam sterilization refrigerator 1 can have a more reasonable structure.

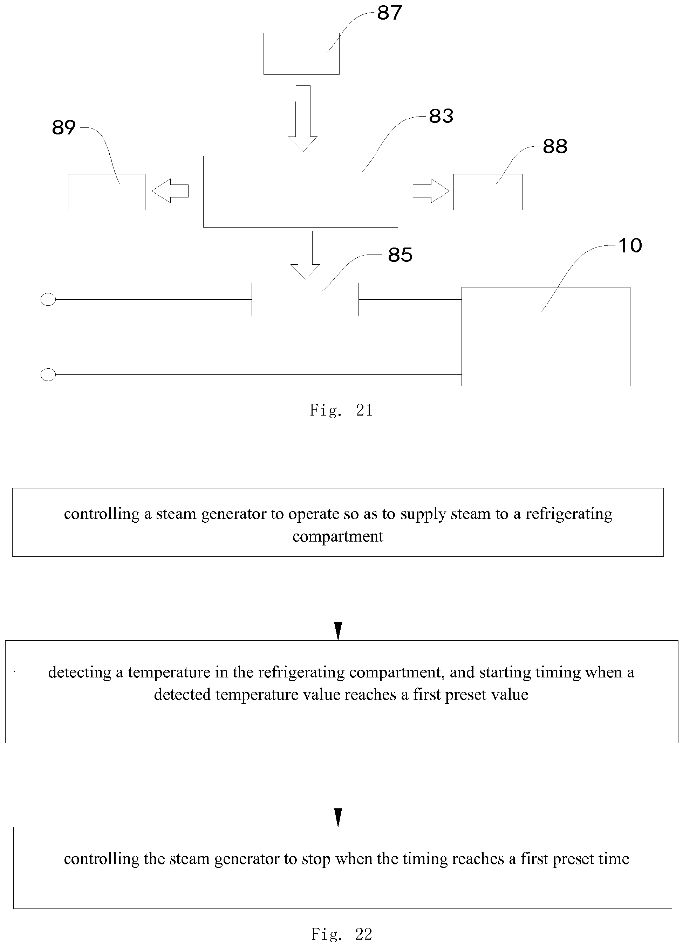

A control method for a steam sterilization refrigerator according to embodiments of the present disclosure will be described below with reference to Figs. 22 to 24. The steam sterilization refrigerator according to embodiments of the present disclosure includes a refrigerator body defining a refrigerating compartment therein, a compressor and a steam generator. Specifically, the steam sterilization refrigerator according to embodiments of the present disclosure may be the steam sterilization refrigerator 1 according to the above embodiments.

The control method for the steam sterilization refrigerator according to embodiments of the present disclosure includes the following steps: controlling the steam generator to operate to supply steam to the refrigerating compartment; detecting a temperature in the refrigerating compartment, and starting timing when a detected temperature value reaches a first preset value; and controlling the steam generator to stop when the timing reaches a first preset time.

In the control method for the steam sterilization refrigerator according to embodiments of the present disclosure, by conveying the steam produced by the steam generator into the refrigerating compartment, the air in the refrigerating compartment can be sterilized, and also bacteria on the wall of the refrigerating compartment and bacteria attached to accessories (such as a storage box and a rack) in the refrigerating compartment can be killed, so as to make the refrigerating compartment cleaner and more hygienic, such that the requirements of the user can be better satisfied.

Advantageously, the first preset value is 60 degrees Celsius to 80 degrees Celsius. That is to say, when the temperature in the refrigerating compartment 21 reaches 60 degrees Celsius to 80 degrees Celsius, the controller 83 starts initial timing to compute the sterilization time. More preferably, the first preset value is 60 degrees Celsius to 70 degrees Celsius. Most preferably, the first preset value is 60 degrees Celsius. Thus, the energy consumption of the steam sterilization refrigerator 1 can be reduced in a case where the sterilizing effect is ensured.

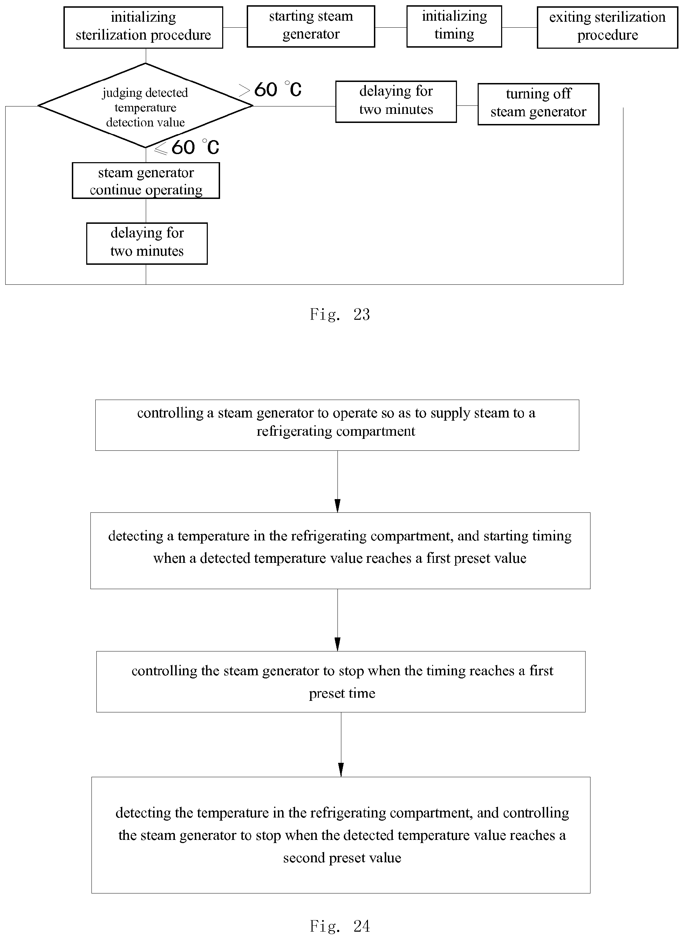

As illustrated in Fig. 23, when the timing (i.e. the initial timing) reaches the first preset time, the controller 83 controls the steam generator 10 to stop. For example, the controller 83 controls the steam generator 10 to stop through the relay 85, that is, the controller 83 controls the steam generator 10 to disconnect from the power source through the relay 85. So far, the sterilization process of the steam sterilization refrigerator 1 is completed.

Advantageously, the first preset time is 10-40 minutes. That is to say, after the temperature in the refrigerating compartment 21 reaches the first preset value, the refrigerating compartment 21 is sterilized for 10-40 minutes.

More preferably, the first preset time is 20-30 minutes. Most preferably, the first preset time is 25 minutes. Thus, in the case where the sterilizing effect is ensured, the energy consumption of the steam sterilization refrigerator 1 can be reduced, and the sterilization time of the refrigerating compartment 21 can be shortened as much as possible, so as to be user-friendly.

As illustrated in Fig. 23, in an embodiment of the present disclosure, the control method further includes comparing the detected temperature value with the first preset value, before the timing reaches the first preset time. In other words, the detected temperature value is compared with the first preset value, before the sterilization of the refrigerating compartment 21 is completed. When the detected temperature value is greater than the first preset value, the steam generator is controlled to stop after a delay of a second preset time; and when the detected temperature value is smaller than or equal to the first preset value, the steam generator is controlled to operate.

Advantageously, the control method for the steam sterilization refrigerator according to embodiments of the present disclosure further includes: comparing the detected temperature value with the first preset value before the timing reaches the first preset time; when the detected temperature value is greater than the first preset value, controlling the steam generator 10 to stop after the delay of the second preset time; and when the detected temperature value is smaller than or equal to the first preset value, controlling the steam generator to operate, and after a delay of a fourth preset time, comparing the detected temperature value with the first preset value again.

Specifically, when the steam produced by the steam generator 10 enters the refrigerating compartment 21 continuously, the temperature in the refrigerating compartment 21 increases continuously. When the temperature in the refrigerating compartment 21 reaches the first preset value, the controller 83 starts the initial timing to compute the sterilization time. Sequentially, the temperature in the refrigerating compartment 21 is greater than the first preset value, that is, the temperature detection value of the temperature detector is greater than the first preset value, and after the delay of the second preset time, the controller 83 controls the steam generator 10 to stop, such that the steam is no longer conveyed into the refrigerating compartment 21.

Due to a heat exchange effect, the temperature in the refrigerating compartment 21 will drop slowly after the steam is no longer conveyed into the refrigerating compartment 21. When the temperature in the refrigerating compartment 21 is smaller than or equal to the first preset value, the controller 83 controls the steam generator 10 to operate, such that the steam is conveyed into the refrigerating compartment 21 again.

After the delay of the fourth preset time, the temperature detection value of the temperature detector is compared with the first preset value again. If the temperature in the refrigerating compartment 21 is greater than the first preset value, after the delay of the second preset time, the controller 83 controls the steam generator 10 to stop. If the temperature in the refrigerating compartment 21 is still smaller than or equal to the first preset value, after the delay of the fourth preset time, the temperature detection value of the temperature detector is compared with the first preset value again, until the temperature in the refrigerating compartment 21 is greater than the first preset value. After the timing (the sterilization time) reaches the first preset time, the temperature detection value of the temperature detector is no longer compared with the first preset value.

Thus, during the sterilization process, the steam generator 10 does not need to operate all the time, and hence the energy consumption of the steam sterilization refrigerator 1 can be reduced in the case where the sterilizing effect is ensured. Moreover, by delaying the turn-off of the steam generator 10, the steam generator 10 can be prevented from being turned on and off frequently, such that the steam generator 10 can be prevented from damage, so as to prolong the service life of the steam generator 10.

Advantageously, the second preset time is 1-3 minutes, and the fourth preset time is 1-3 minutes. More advantageously, the second preset time is 2 minutes, and the fourth preset time is 2 minutes.

In another example of the present disclosure, the control method for the steam sterilization refrigerator according to embodiments of the present disclosure further includes: comparing the temperature in the refrigerating compartment 21 with the first preset value before the timing reaches the first preset time; when the temperature in the refrigerating compartment 21 is greater than the first preset value, controlling the steam generator 10 to stop, and when the temperature in the refrigerating compartment 21 is smaller than or equal to the third preset value, controlling the steam generator to operate, in which the third preset value is smaller than the first preset value.

Thus, during the sterilization process, the steam generator 10 does not need to operate all the time, and hence the energy consumption of the steam sterilization refrigerator 1 can be reduced in the case where the sterilizing effect is ensured. Moreover, the steam generator 10 can be prevented from being turned on and off frequently, such that the steam generator 10 can be prevented from damage, so as to prolong the service life of the steam generator 10. Advantageously, the third preset value is 55 degrees Celsius to 65 degrees Celsius.

As illustrated in Fig. 24, advantageously, the control method for the steam sterilization refrigerator according to embodiments of the present disclosure further includes: detecting a temperature of the steam generator 10, and controlling the steam generator 10 to stop when the temperature of the steam generator 10 reaches the second preset value. Thus, the steam generator 10 can be prevented from the dry heating, and the temperature of the steam generator 10 can be prevented from being too high, such that the safety performance of the steam generator 10 can be significantly improved, and further the safety performance of the steam sterilization refrigerator 1 can be improved.

The second preset value is 160 degrees Celsius to 200 degrees Celsius. Advantageously, the second preset value is 170 degrees Celsius to 190 degrees Celsius. More advantageously, the second preset value is 180 degrees Celsius.

In a specific example of the present disclosure, when the steam generator 10 starts to operate, there is no water in the steam generator 10. The water is supplied to the steam generator 10 when the temperature of the steam generator 10 reaches 50 degrees Celsius to 80 degrees Celsius. Thus, the steam generator 10 can produce the steam with higher temperature, the sterilizing effect can be further improved, and the sterilization time can be further shortened.

Advantageously, the water is supplied into the steam generator 10 at a flow rate of 30 ml/min to 40 ml/min. Thus, a phenomenon of hot water splashing in the steam generator 10 can be prevented.

In a specific example of the present disclosure, after the steam generator 10 is controlled to supply the steam to the refrigerating compartment 21 for the third preset time, and the temperature in the refrigerating compartment 21 is smaller than the first preset value, the steam generator 10 is controlled to stop.

When the steam generator 10 starts to operate (that is the sterilization is started), the user may forget to close the door body 30 or the door body 30 is not closed tightly, in which case the steam entering the refrigerating compartment 21 may leak to the environment, such that the temperature in the refrigerating compartment 21 cannot be raised. If the temperature in the refrigerating compartment 21 is smaller than the first preset value after the third preset time, the steam generator 10 is controlled to stop, such that the steam produced by the steam generator 10 can be prevented from leakage, and also the user can be reminded to close the door body 30 or close the door body 30 tightly.

Advantageously, the third preset time is 35-45 minutes.

In the specification, it is to be understood that terms such as "central," "longitudinal," "lateral," "length," "width," "thickness," "upper," "lower," "front," "rear," "left," "right," "vertical," "horizontal," "top," "bottom," "inner," "outer," "clockwise," "counterclockwise," "axial," "radial" and "circumferential" should be construed to refer to the orientation as then described or as shown in the drawings under discussion. These relative terms are for convenience of description and do not require that the present disclosure be constructed or operated in a particular orientation.

In addition, terms such as "first" and "second" are used herein for purposes of description and are not intended to indicate or imply relative importance or significance. Thus, the feature defined with "first" and "second" may comprise one or more of this feature. In the description of the present disclosure, "a plurality of' means two or more than two, unless specified otherwise.

In the present disclosure, unless specified or limited otherwise, the terms "mounted," "connected," "coupled," "fixed" and the like are used broadly, and may be, for example, fixed connections, detachable connections, or integral connections; may also be mechanical or electrical connections; may also be direct connections or indirect connections via intervening structures; may also be inner communications of two elements. The above terms can be understood by those skilled in the art according to specific situations.

In the present disclosure, unless specified or limited otherwise, a structure in which a first feature is "on" or "below" a second feature may include an embodiment in which the first feature is in direct contact with the second feature, and may also include an embodiment in which the first feature and the second feature are not in direct contact with each other, but are contacted via an additional feature formed therebetween. Furthermore, a first feature "on," "above," or "on top of' a second feature may include an embodiment in which the first feature is right or obliquely "on," "above," or "on top of' the second feature, or just means that the first feature is at a height higher than that of the second feature. While a first feature "below," "under," or "on bottom of' a second feature may include an embodiment in which the first feature is right or obliquely "below," "under," or "on bottom of' the second feature, or just means that the first feature is at a height lower than that of the second feature.

Reference throughout this specification to "an embodiment," "some embodiments," "an example," "a specific example," or "some examples," means that a particular feature, structure, material, or characteristic described in connection with the embodiment or example is included in at least one embodiment or example of the present disclosure. Thus, the appearances of the phrases in various places throughout this specification are not necessarily referring to the same embodiment or example of the present disclosure. Furthermore, the particular features, structures, materials, or characteristics may be combined in any suitable manner in one or more embodiments or examples. In addition, without conflicting, various embodiments or examples or features of various embodiments or examples described in the present specification may be combined by those skilled in the art.

Although explanatory embodiments have been shown and described, it would be appreciated by those skilled in the art that the above embodiments cannot be construed to limit the present disclosure, and changes, alternatives, and modifications can be made in the embodiments without departing from principles and scope of the present disclosure.

## Claims

1. A steam sterilization refrigerator (1), comprising:
a refrigerator body (20) having a refrigerating compartment (21) and a compressor chamber (22) therein;
a door body (30) disposed to the refrigerator body ; and
a steam generator (10,106) disposed to at least one of the refrigerator body and the door body, the steam generator having a steam outlet (1013), the steam outlet being in direct communication with the refrigerating compartment or in indirect communication with the refrigerating compartment via a steam conveying channel (40);
**characterized in that**:
the steam generator is disposed in the compressor chamber, a first side of the steam generator is provided with an inserting part (1014) connected to the refrigerator body in an inserting manner, a second side of the steam generator opposite to the first side is provided with a connecting part (1015), and the connecting part is connected to the refrigerator body;
wherein the steam generator is disposed to one of an upper surface, a left surface, a right surface and a rear surface of the refrigerator body, and wherein the steam generator is elevated from a bottom wall of the compressor chamber.

2. The steam sterilization refrigerator according to claim 1, wherein
the steam conveying channel (40) is formed by a steam conveying tube (41), a first end of the steam conveying tube (41) is connected to the steam outlet (1013), and a second end of the steam conveying tube (41) penetrates a foaming layer (23) of the refrigerator body (20) and extends into the refrigerating compartment (21) or is flush with a wall of the refrigerating compartment (21).

3. The steam sterilization refrigerator according to any one of claims 1-2, further comprising: a water tank (50) detachably disposed to an upper surface of the refrigerator body (20); a flexible connecting tube (51); a first connecting tube (52) having a first end connected to the water tank (50), and a second end detachably fitted in a first end of the flexible connecting tube (51); a conveying pump (53) having a water outlet in communication with a water inlet (1012) of the steam generator (10); and a second connecting tube (54) having a first end fitted in a second end of the flexible connecting tube (51), and a second end connected to a water inlet (1012) of the conveying pump (53); and optionally
wherein an inner circumferential wall of the first end of the flexible connecting tube (51) is provided with a first annular sealing flange (511) and a second annular sealing flange (512), when the second end of the first connecting tube (52) is fitted in the first end of the flexible connecting tube (51), an end surface of the second end of the first connecting tube (52) abuts against the first annular sealing flange (511), and the second annular sealing flange (512) abuts against a circumferential surface of the first connecting tube (52).

4. The steam sterilization refrigerator according to any one of claims 1 to 3, wherein a plurality of first steam holes (211) are provided in a wall of the refrigerating compartment (21) and spaced apart in an up-and-down direction, and the steam conveying channel (40) is formed in the refrigerator body (20) and has a plurality of second steam holes in communication with the plurality of first steam holes (211) in one-to-one correspondence; or
wherein the steam conveying channel (40) is formed by a steam conveying tube, a first portion of the steam conveying tube is disposed in a foaming layer of the refrigerator body (20)opposite to the refrigerating compartment (21), and the first portion of the steam conveying tube is provided with a plurality of second steam holes in communication with a plurality of first steam holes (211) in one-to-one correspondence.

5. The steam sterilization refrigerator according to claim 1, wherein the steam generator is disposed in the refrigerating compartment, the steam sterilization refrigerator further comprises a water tank disposed in the refrigerating compartment, the water tank has a water outlet hermetically connected to a water inlet (1012) of the steam generator, and the steam outlet of the steam generator is in direct communication with the refrigerating compartment; and optionally
wherein the steam generator (10) comprises a furnace body (101) and a water inlet tube (102), a first through hole (1016) is provided in a wall of the furnace body (101), and the water inlet tube is connected to the first through hole; the water tank comprises a water tank body and a water outlet tube, a second through hole is provided in a wall of the water tank body, the water outlet tube is connected to the second through hole, a part of the water inlet tube is fitted in the water outlet tube, and a sealing ring is disposed between the part of the water inlet tube and the water outlet tube; and/or optionally
wherein the part of the water inlet tube is detachably fitted in the water outlet tube, and the water tank further comprises: a valve disc movable between a closed position where an inlet of the water outlet tube is closed and an open position where the inlet of the water outlet tube is open; a valve stem (57) stem disposed in the water outlet tube and connected to the valve disc, the valve stem being driven to move the valve disc from the closed position to the open position when the water inlet tube is inserted into and fitted in the water outlet tube; and an elastic element disposed between the valve stem and the water outlet tube and normally pushing the valve stem and the valve disc towards the closed position; and/or optionally
wherein a wall of the refrigerating compartment is provided with a first limiting member, the water tank is provided with a second limiting member fitted with the first limiting member, and the water tank is spaced apart from the steam generator at a preset distance when the second limiting member is in contact with the first limiting member.

6. The steam sterilization refrigerator according to any preceding claim further comprising: a plurality of racks (24) disposed in the refrigerating compartment (21) and spaced apart from one another in an up-and-down direction, the steam generator (10) and the water tank (50) being disposed between a bottom wall of the refrigerating compartment (21) and a bottom one of the racks; and an air duct cover plate (60) disposed in the refrigerating compartment (21) and located behind the plurality of the racks (24), the steam conveying channel (40) being defined between the air duct cover plate (60) and a wall of the refrigerating compartment (21), the air duct cover plate (60) being provided with a plurality of steam holes (61) in communication with the steam conveying channel, the plurality of steam holes being spaced apart from one another in the up-and-down direction, and each space of a space between adjacent racks and a space between a top one of the racks and a top wall of the refrigerating compartment corresponding to at least one of the steam holes; or
further comprises a plurality of racks disposed in the refrigerating compartment and spaced apart from one another in an up-and-down direction, the steam generator and the water tank being disposed between a bottom wall of the refrigerating compartment and a bottom one of the racks, wherein the steam conveying channel is formed by a steam conveying tube disposed in the refrigerating compartment, a lower port of the steam conveying tube is in communication with the steam outlet, a plurality of steam holes are provided in the steam conveying tube and spaced apart from one another in the up-and-down direction, and each space of a space between adjacent racks and a space between a top one of the racks and a top wall of the refrigerating compartment corresponds to at least one of the steam holes.

7. The steam sterilization refrigerator according to any one of claims 1 to 6, further comprises a storage box (71) disposed in the refrigerating compartment (21) and capable of being pushed into and pulled out of the refrigerating compartment (21), the storage box (71) having a storage chamber (711) adapted to accommodate water and articles, and the storage chamber (711) being in communication with a water inlet (1012) of the steam generator (10) to supply the water in the storage chamber (711) to the steam generator (10); and optionally
further comprising: a water inlet transition member (72) disposed in the refrigerating compartment, a water flow channel (721) being defined in the water inlet transition member (72), and the water flow channel (721) having an inlet (7211) and an outlet (7212); a first water inlet tube (73) disposed to the storage box (71), and having a first end in communication with the storage chamber (711) and a second end in connection with the inlet (7211) of the water inlet transition member (72) when the storage box (71) is located in the refrigerating compartment (21); and a second water inlet tube (74) having a first end connected to the outlet of the water inlet transition member, and a second end in communication with the water inlet of the steam generator.

8. The steam sterilization refrigerator according to claim 1, further comprising:
an air duct component (91), a ventilating channel (911) being defined in the air duct component (91) or between the air duct component (91) and a wall of the refrigerating compartment (21), the ventilating channel being in communication with each of the refrigerating compartment and the steam outlet, and the steam conveying channel being formed by the ventilating channel; an evaporator disposed in the ventilating channel; and a fan disposed in the ventilating channel to convey steam and cold air into the refrigerating compartment; and optionally
wherein the refrigerator body has a refrigerating chamber and a freezing chamber therein, and the ventilating channel is in communication with each of the refrigerating chamber, the freezing chamber and the steam outlet; and/or optionally
further comprises a steam connecting tube having a first end connected to the steam outlet, and a second end extending into the ventilating channel and opening towards the evaporator.

9. The steam sterilization refrigerator according to any one of claims 1 to 8, wherein the steam generator comprises a furnace body (101) and an electric heater (104), an accommodating cavity (1011) for accommodating water is defined in the furnace body therein, the accommodating cavity has a water inlet and the steam outlet, the electric heater is adapted to be connected to a power source through a power cord (95), and the steam sterilization refrigerator further comprises:
a conveying pump having a water outlet in communication with the water inlet of the steam generator;
a temperature control protector disposed to an outer surface of the furnace body and configured to sense a temperature of the outer surface of the furnace body, the temperature control protector being connected to the power cord and automatically turning off when the temperature of the outer surface of the furnace body is greater than or equal to a preset temperature;
a temperature sensor disposed to the outer surface of the furnace body and configured to sense the temperature of the outer surface of the furnace body; and
a controller connected to the temperature sensor and the electric heater and controlling the electric heater to stop heating when the temperature of the outer surface of the furnace body is greater than or equal to the preset temperature.

10. The steam sterilization refrigerator according to claim 9, wherein the controller (83) is connected to an electric motor of the conveying pump (53) of the conveying pump to monitor a working current of the electric motor of the conveying pump, the steam sterilization refrigerator further comprises a first indicator light (841) and a second indicator light (842), each of the first indicator light and the second indicator light being connected to the controller, wherein when the working current of the electric motor of the conveying pump is within a preset range and the electric heater stops heating, the controller controls the first indicator light to glow or flicker, and when the working current of the electric motor of the conveying pump exceeds the preset range and the electric heater stops heating, the controller controls the second indicator light to glow or flicker; or
further comprises a voice reminder means (86) connected to the controller, wherein when a working current of an electric motor of the conveying pump is within a preset range and the electric heater stops heating, the controller controls the voice reminder means to give out a first reminding voice, and when the working current of the electric motor of the conveying pump exceeds the preset range and the electric heater stops heating, the controller controls the voice reminder to give out a second reminding voice.

11. The steam sterilization refrigerator according to any one of claims 1-10, further comprising:
a water level gauge (87) configured to detect a water level in a water tank (50);
a water-shortage indicator light (88) and a full-water indicator (89) light both disposed in the refrigerating compartment (21); and
a controller (83) connected to each of the water level gauge, the water-shortage indicator light, the full-water indicator light and the steam generator, wherein the controller (83) controls the water-shortage indicator light to glow or flicker when a water level detection value of the water level gauge is smaller than a first preset value, and controls the full-water indicator light to glow or flicker when the water level detection value of the water level gauge is greater than a second preset value.

12. A control method for a steam sterilization refrigerator (1) according to any of the preceding claims, wherein the steam sterilization refrigerator comprises a refrigerator body (20) defining a refrigerating compartment (21) therein, a compressor (22) and a steam generator (10), and the control method comprises steps:
controlling the steam generator (10) to operate to supply steam to the refrigerating compartment (21);
detecting a temperature in the refrigerating compartment (21), and starting timing when a detected temperature value reaches a first preset value;
controlling the steam generator (10) to stop when the timing reaches a first preset time; and
detecting the temperature in the refrigerating compartment (21), and controlling the steam generator (10) to stop when the detected temperature value reaches a second preset value.

13. The control method according to claim 12, wherein the first preset value is 60-80 degrees Celsius, and the first preset time is 10-40 minutes; or
further comprising: comparing the detected temperature value with the first preset value before the timing reaches the first preset time, controlling the steam generator (10) to stop after a delay of a second preset time when the detected temperature value is greater than the first preset value, and controlling the steam generator (10) to operate when the detected temperature value is smaller than or equal to the first preset value; or
wherein water is supplied to the steam generator (10) when a temperature of the steam generator (10) reaches 50 degrees Celsius to 80 degrees Celsius.

14. The control method according to claim 12,
wherein the first preset value is 60-80 degrees Celsius, the second preset value is 160-200 degrees Celsius, and the first preset time is 10-40 minutes.

15. The control method according to any one of claim 12 to 14, wherein after the steam generator (10) is controlled to supply steam to the refrigerating compartment (21) for a third preset time, and when the temperature in the refrigerating compartment is smaller than the first preset value, the steam generator is controlled to stop; and optionally
wherein the third preset time is 35-45 minutes.

## Patentansprüche

1. Kühlschrank mit Dampfsterilisation (1), der Folgendes aufweist:
einen Kühlschrankkörper (20) mit einem Kühlfach (21) und einer Kompressorkammer (22) in ihm;
einen Türkörper (30), der am Kühlschrankkörper angeordnet ist; und
einen Dampfgenerator (10, 106), der an wenigstens einem von dem Kühlschrankkörper und dem Türkörper angeordnet ist, wobei der Dampfgenerator einen Dampfauslass (1013) hat, wobei der Dampfauslass in direkter Verbindung mit dem Kühlfach oder über einen Dampfförderkanal (40) in indirekter Verbindung mit dem Kühlfach steht;
**dadurch gekennzeichnet, dass**:
der Dampfgenerator in der Kompressorkammer angeordnet ist, eine erste Seite des Dampfgenerators mit einem Einsteckteil (1014) versehen ist, der mit dem Kühlschrankkörper auf eine Einsteckweise verbunden ist, eine der ersten Seite entgegengesetzte zweite Seite des Dampfgenerators mit einem Verbindungsteil (1015) versehen ist und der Verbindungsteil mit dem Kühlschrankkörper verbunden ist;
wobei der Dampfgenerator an einer von einer oberen Oberfläche, einer linken Oberfläche, einer rechten Oberfläche und einer hinteren Oberfläche des Kühlschrankkörpers angeordnet ist und wobei der Dampfgenerator höher als eine untere Wand der Kompressorkammer liegt.

2. Kühlschrank mit Dampfsterilisation nach Anspruch 1, wobei
der Dampfförderkanal (40) von einer Dampfförderröhre (41) gebildet wird, wobei ein erstes Ende der Dampfförderröhre (41) mit dem Dampfauslass (1013) verbunden ist und ein zweites Ende der Dampfförderröhre (41) eine Verschäumungsschicht (23) des Kühlschrankkörpers (20) durchdringt und sich in das Kühlfach (21) hinein erstreckt oder mit einer Wand des Kühlfachs (21) bündig ist.

3. Kühlschrank mit Dampfsterilisation nach einem der Ansprüche 1 bis 2, der ferner Folgendes aufweist: einen Wassertank (50), der abnehmbar an einer oberen Oberfläche des Kühlschrankkörpers (20) angeordnet ist; eine flexible Verbindungsröhre (51); eine erste Verbindungsröhre (52), die ein mit dem Wassertank (50) verbundenes erstes Ende und ein abnehmbar in ein erstes Ende der flexiblen Verbindungsröhre (51) eingepasstes zweites Ende hat; eine Förderpumpe (53), die einen mit einem Wassereinlass (1012) des Dampfgenerators (10) in Verbindung stehenden Wasserauslass hat; und eine zweite Verbindungsröhre (54), die ein in ein zweites Ende der flexiblen Verbindungsröhre (51) eingepasstes erstes Ende und ein mit einem Wassereinlass (1012) der Förderpumpe (53) verbundenes zweites Ende hat; und wahlweise
wobei eine innere Umfangswand des ersten Endes der flexiblen Verbindungsröhre (51) mit einem ersten ringförmigen Dichtungsflansch (511) und einem zweiten ringförmigen Dichtungsflansch (512) versehen ist, wenn das zweite Ende der ersten Verbindungsröhre (52) in das erste Ende der flexiblen Verbindungsröhre (51) eingepasst ist, eine Endfläche des zweiten Endes der ersten Verbindungsröhre (52) am ersten ringförmigen Dichtungsflansch (511) in Anlage ist und der zweite ringförmige Dichtungsflansch (512) an einer Umfangsfläche der ersten Verbindungsröhre (52) in Anlage ist.

4. Kühlschrank mit Dampfsterilisation nach einem der Ansprüche 1 bis 3, wobei mehrere erste Dampflöcher (211) in einer Wand des Kühlfachs (21) bereitgestellt sind und in einer Aufwärts- und Abwärtsrichtung voneinander beabstandet sind und der Dampfförderkanal (40) im Kühlschrankkörper (20) gebildet ist und mehrere zweite Dampflöcher hat, die mit den mehreren ersten Dampflöchern (211) in Eins-zu-Eins-Entsprechung in Verbindung sind; oder
wobei der Dampfförderkanal (40) von einer Dampfförderröhre gebildet wird, ein erster Teil der Dampfförderröhre in einer Verschäumungsschicht des Kühlschrankkörpers (20), dem Kühlfach (21) entgegengesetzt, angeordnet ist und der erste Teil der Dampfförderröhre mit mehreren zweiten Dampflöchern versehen ist, die mit mehreren ersten Dampflöchern (211) in Eins-zu-Eins-Entsprechung in Verbindung stehen.

5. Kühlschrank mit Dampfsterilisation nach Anspruch 1, wobei der Dampfgenerator im Kühlfach angeordnet ist, der Kühlschrank mit Dampfsterilisation ferner einen im Kühlfach angeordneten Wassertank aufweist, der Wassertank einen Wasserauslass hat, der hermetisch mit einem Wassereinlass (1012) des Dampfgenerators verbunden ist und der Dampfauslass des Dampfgenerators mit dem Kühlfach in direkter Verbindung steht; und wahlweise
wobei der Dampfgenerator (10) einen Ofenkörper (101) und eine Wassereinlassröhre (102) aufweist, ein erstes Durchgangsloch (1016) in einer Wand des Ofenkörpers (101) bereitgestellt ist und die Wassereinlassröhre mit dem ersten Durchgangsloch verbunden ist; der Wassertank einen Wassertankkörper und eine Wasserauslassröhre aufweist, ein zweites Durchgangsloch in einer Wand des Wassertankkörpers bereitgestellt ist, die Wasserauslassröhre mit dem zweiten Durchgangsloch verbunden ist, ein Teil der Wassereinlassröhre in die Wasserauslassröhre eingepasst ist und ein Dichtungsring zwischen dem Teil der Wassereinlassröhre und der Wasserauslassröhre angeordnet ist; und/oder wahlweise
wobei der Teil der Wassereinlassröhre abnehmbar in die Wasserauslassröhre eingepasst ist und der Wassertank ferner Folgendes aufweist: einen zwischen einer geschlossenen Stellung, in der ein Einlass der Wasserauslassröhre geschlossen ist, und einer offenen Stellung, in der der Einlass der Wasserauslassröhre offen ist, bewegbaren Ventilteller; einen Ventilschaft (57), der in der Wasserauslassröhre angeordnet ist und mit dem Ventilteller verbunden ist, wobei der Ventilschaft angetrieben wird, um den Ventilteller von der geschlossenen Stellung in die offene Stellung zu bewegen, wenn die Wassereinlassröhre in die Wasserauslassröhre eingefügt und eingepasst wird; und ein elastisches Element, das zwischen dem Ventilschaft und der Wasserauslassröhre angeordnet ist und normalerweise den Ventilschaft und den Ventilteller in Richtung auf die geschlossene Stellung schiebt; und/oder wahlweise
wobei eine Wand des Kühlfachs mit einem ersten Begrenzungselement versehen ist, der Wassertank mit einem zweiten Begrenzungselement, das mit dem ersten Begrenzungselement zusammengepasst ist, versehen ist und der Wassertank in einem vorgegebenen Abstand vom Dampfgenerator beabstandet ist, wenn das zweite Begrenzungselement mit dem ersten Begrenzungselement in Kontakt ist.

6. Kühlschrank mit Dampfsterilisation nach einem der vorhergehenden Ansprüche, der ferner Folgendes aufweist: mehrere Ablagen (24), die im Kühlfach (21) angeordnet sind und in einer Aufwärts- und Abwärtsrichtung voneinander beabstandet sind, wobei der Dampfgenerator (10) und der Wassertank (50) zwischen einer unteren Wand des Kühlfachs (21) und einer unteren der Ablagen angeordnet sind; und eine Luftkanalabdeckplatte (60), die im Kühlfach (21) angeordnet ist und hinter den mehreren Ablagen (24) liegt, wobei der Dampfförderkanal (40) zwischen der Luftkanalabdeckplatte (60) und einer Wand des Kühlfachs (21) definiert wird, wobei die Luftkanalabdeckplatte (60) mit mehreren Dampflöchern (61) versehen ist, die mit dem Dampfförderkanal in Verbindung stehen, wobei die mehreren Dampflöcher in der Aufwärts- und Abwärtsrichtung voneinander beabstandet sind und jeder Zwischenraum von einem Zwischenraum zwischen benachbarten Ablagen und einem Zwischenraum zwischen einer oberen der Ablagen und einer oberen Wand des Kühlfachs wenigstens einem der Dampflöcher entspricht; oder
ferner mehrere Ablagen aufweist, die im Kühlfach angeordnet und in einer Aufwärts- und Abwärtsrichtung voneinander beabstandet sind, wobei der Dampfgenerator und der Wassertank zwischen einer unteren Wand des Kühlfachs und einer unteren der Ablagen angeordnet ist, wobei der Dampfförderkanal von einer im Kühlfach angeordneten Dampfförderröhre gebildet wird, ein unterer Anschluss der Dampfförderröhre mit dem Dampfauslass in Verbindung steht, in der Dampfförderröhre mehrere Dampflöcher bereitgestellt und in der Aufwärts- und Abwärtsrichtung voneinander beabstandet sind und jeder Zwischenraum von einem Zwischenraum zwischen benachbarten Ablagen und einem Zwischenraum zwischen einer oberen der Ablagen und einer oberen Wand des Kühlfachs wenigstens einem der Dampflöcher entspricht.

7. Kühlschrank mit Dampfsterilisation nach einem der Ansprüche 1 bis 6, der einen Aufbewahrungskasten (71) aufweist, der im Kühlfach (21) angeordnet ist und in das bzw. aus dem Kältefach (21) hineingeschoben und herausgezogen werden kann, wobei der Aufbewahrungskasten (71) eine Aufbewahrungskammer (711) hat, die zur Aufnahme von Wasser und Artikeln geeignet ist, und die Aufbewahrungskammer (711) mit einem Wassereinlass (1012) des Dampfgenerators (10) zum Zuführen des Wassers in der Aufbewahrungskammer (711) zum Dampfgenerator (10) in Verbindung steht; und wahlweise
ferner Folgendes aufweist: ein Wassereinlassübergangsstück (72), das im Kühlfach angeordnet ist, wobei im Wassereinlassübergangsstück (72) ein Wasserdurchflusskanal (721) definiert wird und der Wasserdurchflusskanal (721) einen Einlass (7211) und einen Auslass (7212) hat; eine erste Wassereinlassröhre (73), die am Aufbewahrungskasten (71) angeordnet ist und ein erstes Ende, das mit der Aufbewahrungskammer (711) in Verbindung steht, und ein zweites Ende, das mit dem Einlass (7211) des Wassereinlassübergangsstücks (72) in Verbindung steht, wenn der Aufbewahrungskasten (71) sich im Kühlfach (21) befindet, hat; und eine zweite Wassereinlassröhre (74), die ein erstes Ende, das mit dem Auslass des Wassereinlassübergangsstücks verbunden ist, und ein zweites Ende, das mit dem Wassereinlass des Dampfgenerators in Verbindung steht, hat.

8. Kühlschrank mit Dampfsterilisation nach Anspruch 1, der ferner Folgendes aufweist:
eine Luftkanalkomponente (91), einen Lüftungskanal (911), der in der Luftkanalkomponente (91) oder zwischen der Luftkanalkomponente (91) und einer Wand des Kühlfachs (21) definiert wird, wobei der Lüftungskanal mit jedem von dem Kühlfach und dem Dampfauslass in Verbindung steht und der Dampfförderkanal von dem Lüftungskanal gebildet wird; einen im Lüftungskanal angeordneten Verdampfer; und einen im Lüftungskanal angeordneten Lüfter zum Fördern von Dampf und Kaltluft in das Kühlfach; und wahlweise
wobei der Kühlschrankkörper eine Kühlkammer und eine Gefrierkammer in ihm hat und der Lüftungskanal mit jeder von der Kühlkammer, der Gefrierkammer und dem Dampfauslass in Verbindung steht; und/oder wahlweise
ferner eine Dampfverbindungsröhre aufweist, die ein erstes Ende, das mit dem Dampfauslass verbunden ist, und ein zweites Ende, das sich in den Lüftungskanal erstreckt und zum Verdampfer mündet, hat.

9. Kühlschrank mit Dampfsterilisation nach einem der Ansprüche 1 bis 8, wobei der Dampfgenerator einen Ofenkörper (101) und eine elektrische Heizung (104) aufweist, im Ofenkörper darin ein Aufnahmehohlraum (1011) zur Aufnahme von Wasser definiert wird, der Aufnahmehohlraum einen Wassereinlass und einen Dampfauslass hat, die elektrische Heizung geeignet ist, um durch ein Stromkabel (95) mit einer Stromquelle verbunden zu sein, und der Kühlschrank mit Dampfsterilisation ferner Folgendes aufweist:
eine Förderpumpe, die einen Wasserauslass hat, der mit dem Wassereinlass des Dampfgenerators in Verbindung steht;
eine Temperaturregelungsschutzeinrichtung, die an einer Außenfläche des Ofenkörpers angeordnet ist und zum Erfassen einer Temperatur der Außenfläche des Ofenkörpers konfiguriert ist, wobei die Temperaturregelungsschutzeinrichtung mit dem Stromkabel verbunden ist und automatisch abschaltet, wenn die Temperatur der Außenfläche des Ofenkörpers größer oder gleich einer vorgegebenen Temperatur ist;
einen Temperaturfühler, der an der Außenfläche des Ofenkörpers angeordnet ist und zum Erfassen der Temperatur der Außenfläche des Ofenkörpers konfiguriert ist; und
eine Steuereinheit, die mit dem Temperatursensor und der elektrischen Heizung verbunden ist und die elektrische Heizung steuert, um das Heizen abzustellen, wenn die Temperatur der Außenfläche des Ofenkörpers größer oder gleich der vorgegebenen Temperatur ist.

10. Kühlschrank mit Dampfsterilisation nach Anspruch 9, wobei die Steuereinheit (83) mit einem Förderpumpen-Elektromotor (53) der Förderpumpe verbunden ist, um einen Arbeitsstrom des Förderpumpen-Elektromotors zu überwachen, wobei der Kühlschrank mit Dampfsterilisation ferner eine erste Anzeigeleuchte (841) und eine zweite Anzeigeleuchte (842) aufweist, die erste Anzeigeleuchte und die zweite Anzeigeleuchte dabei jeweils mit der Steuereinheit verbunden sind, wobei, wenn der Arbeitsstrom des Förderpumpen-Elektromotors innerhalb eines vorgegebenen Bereichs ist und die elektrische Heizung zu heizen aufhört, die Steuereinheit die erste Anzeigeleuchte steuert, so dass sie leuchtet oder flackert, und, wenn der Arbeitsstrom des Förderpumpen-Elektromotors den vorgegebenen Bereich übersteigt und die elektrische Heizung zu heizen aufhört, die Steuereinheit die zweite Anzeigeleuchte steuert, so dass sie leuchtet oder flackert; oder
ferner ein mit der Steuereinheit verbundenes Sprachhinweismittel (86) aufweist, wobei, wenn ein Arbeitsstrom eines Förderpumpen-Elektromotors sich innerhalb eines vorgegebenen Bereichs befindet und die elektrische Heizung zu heizen aufhört, die Steuereinheit das Sprachhinweismittel steuert, so dass es einen ersten Sprachhinweis ausgibt, und, wenn der Arbeitsstrom des Förderpumpen-Elektromotors den vorgegebenen Bereichs übersteigt und die elektrische Heizung zu heizen aufhört, die Steuereinheit den Sprachhinweisgeber steuert, so dass er einen zweiten Sprachhinweis ausgibt.

11. Kühlschrank mit Dampfsterilisation nach einem der Ansprüche 1 bis 10, der ferner Folgendes aufweist:
einen Wasserstandsmesser (87), der zum Ermitteln eines Wasserstands in einem Wassertank (50) konfiguriert ist;
eine Wassermangelanzeigeleuchte (88) und eine Wasservollstandsanzeige- (89) -leuchte, die beide im Kühlfach (21) angeordnet sind; und
eine Steuereinheit (83), die mit jedem von dem Wasserstandsmesser, der Wassermangelanzeigeleuchte, der Wasservollstandsanzeigeleuchte und dem Dampfgenerator verbunden ist, wobei die Steuereinheit (83) die Wassermangelanzeigeleuchte steuert, so dass sie leuchtet oder flackert, wenn ein Wasserstanderkennungswert des Wasserstandsmessers kleiner als ein erster vorgegebener Wert ist, und die Wasservollstandsanzeigeleuchte steuert, so dass sie leuchtet oder flackert, wenn der Wasserstanderkennungswert des Wasserstandsmessers größer als ein zweiter vorgegebener Wert ist.

12. Steuerungsverfahren für einen Kühlschrank mit Dampfsterilisation (1) nach einem der vorhergehenden Ansprüche, wobei der Kühlschrank mit Dampfsterilisation einen Kühlschrankkörper (20), der darin ein Kühlfach (21) definiert, einen Kompressor (22) und einen Dampfgenerator (10) aufweist und das Steuerungsverfahren die folgenden Schritte aufweist:
Steuern des Dampfgenerators (10), so dass er zum Zuführen von Dampf zum Kühlfach (21) betrieben wird;
Ermitteln einer Temperatur im Kühlfach (21) und Starten einer Zeitmessung, wenn ein ermittelter Temperaturwert einen ersten vorgegebenen Wert erreicht;
Steuern des Dampfgenerators (10), so dass er abgestellt wird, wenn die Zeitmessung eine erste vorgegebene Zeit erreicht; und
Ermitteln der Temperatur im Kühlfach (21) und Steuern des Dampfgenerators (10), so dass er abgestellt wird, wenn der ermittelte Temperaturwert einen zweiten vorgegebenen Wert erreicht.

13. Steuerungsverfahren nach Anspruch 12, wobei der erste vorgegebene Wert 60 - 80 Grad Celsius ist und die erste vorgegebene Zeit 10 - 40 Minuten ist; oder
das ferner Folgendes aufweist: Vergleichen des ermittelten Temperaturwerts mit dem ersten vorgegebenen Wert, bevor die Zeitmessung den ersten vorgegebenen Wert erreicht, Steuern des Dampfgenerators (10), so dass er nach einer Verzögerung von einer zweiten vorgegebenen Zeit abgestellt wird, wenn der ermittelte Temperaturwert größer als der erste vorgegebene Wert ist, und Steuern des Dampfgenerators (10), so dass er betrieben wird, wenn der ermittelte Temperaturwert kleiner oder gleich dem ersten vorgegebenen Wert ist; oder
wobei dem Dampfgenerator (10) Wasser zugeführt wird, wenn eine Temperatur des Dampfgenerators (10) 50 Grad Celsius bis 80 Grad Celsius erreicht.

14. Steuerungsverfahren nach Anspruch 12,
wobei der erste vorgegebene Wert 60 - 80 Grad Celsius ist, der zweite vorgegebene Wert 160 - 200 Grad Celsius ist und die erste vorgegebene Zeit 10 - 40 Minuten ist.

15. Steuerungsverfahren nach einem der Ansprüche 12 bis 14, wobei der Dampfgenerator, nach dem Steuern des Dampfgenerators (10), so dass er dem Kühlfach (21) eine dritte vorgegebene Zeit lang Dampf zuführt, und wenn die Temperatur im Kühlfach kleiner als der erste vorgegebene Wert ist, gesteuert wird, so dass er abgestellt wird; und wahlweise
wobei die dritte vorgegebene Zeit 35 -45 Minuten ist.

## Revendications

1. Réfrigérateur de stérilisation à la vapeur (1), comprenant :
un corps de réfrigérateur (20) ayant un compartiment de réfrigération (21) et une chambre de compresseur (22) dans celui-ci ;
un corps de porte (30) disposé sur le corps de réfrigérateur ; et
un générateur de vapeur (10, 106) disposé sur au moins l'un du corps de réfrigérateur et du corps de porte, le générateur de vapeur ayant une sortie de vapeur (1013), la sortie de vapeur étant en communication directe avec le compartiment de réfrigération ou en communication indirecte avec le compartiment de réfrigération par l'intermédiaire d'un canal de transport de vapeur (40) ;
**caractérisé en ce que** :
le générateur de vapeur est disposé dans la chambre de compresseur, un premier côté du générateur de vapeur est doté d'une pièce d'insertion (1014) reliée au corps de réfrigérateur par insertion, un deuxième côté du générateur de vapeur opposé au premier côté est doté d'une pièce de connexion (1015), et la pièce de connexion est reliée au corps de réfrigérateur ;
où le générateur de vapeur est disposé sur l'une d'une surface supérieure, d'une surface gauche, d'une surface droite et d'une surface arrière du corps de réfrigérateur, et où le générateur de vapeur est élevé par rapport à une paroi inférieure de la chambre de compresseur.

2. Réfrigérateur de stérilisation à la vapeur selon la revendication 1, où
le canal de transport de vapeur (40) est formé par un tube de transport de vapeur (41), une première extrémité du tube de transport de vapeur (41) est reliée à la sortie de vapeur (1013), et une deuxième extrémité du tube de transport de vapeur (41) pénètre dans une couche de moussage (23) du corps de réfrigérateur (20) et s'étend jusque dans le compartiment de réfrigération (21) ou est de niveau avec une paroi du compartiment de réfrigération (21).

3. Réfrigérateur de stérilisation à la vapeur selon l'une quelconque des revendications 1 à 2, comprenant en outre : un réservoir d'eau (50) disposé de manière amovible sur une surface supérieure du corps de réfrigérateur (20) ; un tube de connexion souple (51) ; un premier tube de connexion (52) ayant une première extrémité reliée au réservoir d'eau (50), et une deuxième extrémité montée de manière amovible dans une première extrémité du tube de connexion souple (51) ; une pompe de transport (53) ayant une sortie d'eau en communication avec une entrée d'eau (1012) du générateur de vapeur (10) ; et un deuxième tube de connexion (54) ayant une première extrémité montée dans une deuxième extrémité du tube de connexion souple (51), et une deuxième extrémité reliée à une entrée d'eau (1012) de la pompe de transport (53) ; et facultativement
où une paroi circonférentielle interne de la première extrémité du tube de connexion souple (51) est dotée d'une première bride d'étanchéité annulaire (511) et d'une deuxième bride d'étanchéité annulaire (512), lorsque la deuxième extrémité du premier tube de connexion (52) est montée dans la première extrémité du tube de connexion souple (51), une surface d'extrémité de la deuxième extrémité du premier tube de connexion (52) est en butée contre la première bride d'étanchéité annulaire (511), et la deuxième bride d'étanchéité annulaire (512) est en butée contre une surface circonférentielle du premier tube de connexion (52).

4. Réfrigérateur de stérilisation à la vapeur selon l'une quelconque des revendications 1 à 3, où une pluralité de premiers trous de vapeur (211) sont disposés dans une paroi du compartiment de réfrigération (21) et espacés selon une direction haut et bas, et le canal de transport de vapeur (40) est formé dans le corps de réfrigérateur (20) et a une pluralité de deuxièmes trous de vapeur en communication avec la pluralité de premiers trous de vapeur (211) en correspondance un à un ; ou
où le canal de transport de vapeur (40) est formé par un tube de transport de vapeur, une première partie du tube de transport de vapeur est disposée dans une couche de moussage du corps de réfrigérateur (20) opposée au compartiment de réfrigération (21), et la première partie du tube de transport de vapeur est dotée d'une pluralité de deuxièmes trous de vapeur en communication avec une pluralité de premiers trous de vapeur (211) en correspondance un à un.

5. Réfrigérateur de stérilisation à la vapeur selon la revendication 1, où le générateur de vapeur est disposé dans le compartiment de réfrigération, le réfrigérateur de stérilisation à la vapeur comprend en outre un réservoir d'eau disposé dans le compartiment de réfrigération, le réservoir d'eau a une sortie d'eau reliée hermétiquement à une entrée d'eau (1012) du générateur de vapeur, et la sortie de vapeur du générateur de vapeur est en communication directe avec le compartiment de réfrigération ; et facultativement
où le générateur de vapeur (10) comprend un corps de four (101) et un tube d'entrée d'eau (102), un premier trou traversant (1016) est prévu dans une paroi du corps de four (101), et le tube d'entrée d'eau est relié au premier trou traversant ; le réservoir d'eau comprend un corps de réservoir d'eau et un tube de sortie d'eau, un deuxième trou traversant est prévu dans une paroi du corps de réservoir d'eau, le tube de sortie d'eau est relié au deuxième trou traversant, une partie du tube d'entrée d'eau est montée dans le tube de sortie d'eau, et une bague d'étanchéité est disposée entre la partie du tube d'entrée d'eau et le tube de sortie d'eau ; et/ou facultativement
où la partie du tube d'entrée d'eau est montée de manière amovible dans le tube de sortie d'eau, et le réservoir d'eau comprend en outre : un disque de soupape mobile entre une position fermée où une entrée du tube de sortie d'eau est fermée et une position ouverte où l'entrée du tube de sortie d'eau est ouverte ; une tige de soupape (57) disposée dans le tube de sortie d'eau et reliée au disque de soupape, la tige de soupape étant entraînée pour déplacer le disque de soupape de la position fermée vers la position ouverte lorsque le tube d'entrée d'eau est inséré et monté dans le tube de sortie d'eau ; et un élément élastique disposé entre la tige de soupape et le tube de sortie d'eau et poussant normalement la tige de soupape et le disque de soupape en direction de la position fermée ; et/ou facultativement
où une paroi du compartiment de réfrigération est dotée d'un premier élément limiteur, le réservoir d'eau est doté d'un deuxième élément limiteur monté avec le premier élément limiteur, et le réservoir d'eau est espacé du générateur de vapeur à une distance prédéfinie lorsque le deuxième élément limiteur est en contact avec le premier élément limiteur.

6. Réfrigérateur de stérilisation à la vapeur selon l'une quelconque des revendications précédentes comprenant en outre : une pluralité d'étagères (24) disposées dans le compartiment de réfrigération (21) et espacées les unes des autres selon une direction haut et bas, le générateur de vapeur (10) et le réservoir d'eau (50) étant disposés entre une paroi inférieure du compartiment de réfrigération (21) et une étagère inférieure des étagères ; et une plaque de recouvrement de conduit d'air (60) disposée dans le compartiment de réfrigération (21) et située derrière la pluralité des étagères (24), le canal de transport de vapeur (40) étant défini entre la plaque de recouvrement de conduit d'air (60) et une paroi du compartiment de réfrigération (21), la plaque de recouvrement de conduit d'air (60) étant dotée d'une pluralité de trous de vapeur (61) en communication avec le canal de transport de vapeur, la pluralité de trous de vapeur étant espacés les uns des autres selon la direction haut et bas, et chaque espace d'un espace entre des étagères adjacentes et d'un espace entre une étagère supérieure des étagères et une paroi supérieure du compartiment de réfrigération correspondant à au moins un des trous de vapeur ; ou
comprend en outre une pluralité d'étagères disposées dans le compartiment de réfrigération et espacées les unes des autres selon une direction haut et bas, le générateur de vapeur et le réservoir d'eau étant disposés entre une paroi inférieure du compartiment de réfrigération et une étagère inférieure des étagères, où le canal de transport de vapeur est formé par un tube de transport de vapeur disposé dans le compartiment de réfrigération, un orifice inférieur du tube de transport de vapeur est en communication avec la sortie de vapeur, une pluralité de trous de vapeur sont disposés dans le tube de transport de vapeur et espacés les uns des autres selon la direction haut et bas, et chaque espace d'un espace entre des étagères adjacentes et d'un espace entre une étagère supérieure des étagères et une paroi supérieure du compartiment de réfrigération correspond à au moins un des trous de vapeur.

7. Réfrigérateur de stérilisation à la vapeur selon l'une quelconque des revendications 1 à 6 comprend en outre une boîte de stockage (71) disposée dans le compartiment de réfrigération (21) et pouvant être poussée dans le compartiment de réfrigération (21) et tirée hors de celui-ci, la boîte de stockage (71) ayant une chambre de stockage (711) adaptée pour recevoir de l'eau et des articles, et la chambre de stockage (711) étant en communication avec une entrée d'eau (1012) du générateur de vapeur (10) pour amener l'eau dans la chambre de stockage (711) au générateur de vapeur (10) ; et facultativement
comprenant en outre : un élément de transition d'entrée d'eau (72) disposé dans le compartiment de réfrigération, un canal d'écoulement d'eau (721) étant défini dans l'élément de transition d'entrée d'eau (72), et le canal d'écoulement d'eau (721) ayant une entrée (7211) et une sortie (7212) ; un premier tube d'entrée d'eau (73) disposé sur la boîte de stockage (71), et ayant une première extrémité en communication avec la chambre de stockage (711) et une deuxième extrémité en connexion avec l'entrée (7211) de l'élément de transition d'entrée d'eau (72) lorsque la boîte de stockage (71) est située dans le compartiment de réfrigération (21) ; et un deuxième tube d'entrée d'eau (74) ayant une première extrémité reliée à la sortie de l'élément de transition d'entrée d'eau, et une deuxième extrémité en communication avec l'entrée d'eau du générateur de vapeur.

8. Réfrigérateur de stérilisation à la vapeur selon la revendication 1, comprenant en outre :
un composant de conduit d'air (91), un canal de ventilation (911) étant défini dans le composant de conduit d'air (91) ou entre le composant de conduit d'air (91) et une paroi du compartiment de réfrigération (21), le canal de ventilation étant en communication avec chacun du compartiment de réfrigération et de la sortie de vapeur, et le canal de transport de vapeur étant formé par le canal de ventilation ; un évaporateur disposé dans le canal de ventilation ; et un ventilateur disposé dans le canal de ventilation pour transporter de la vapeur et de l'air froid jusque dans le compartiment de réfrigération ; et facultativement
où le corps de réfrigérateur a une chambre de réfrigération et une chambre de congélation dans celui-ci, et le canal de ventilation est en communication avec chacune de la chambre de réfrigération, de la chambre de congélation et de la sortie de vapeur ; et/ou facultativement
comprend en outre un tube de connexion de vapeur ayant une première extrémité reliée à la sortie de vapeur, et une deuxième extrémité s'étendant jusque dans le canal de ventilation et s'ouvrant en direction de l'évaporateur.

9. Réfrigérateur de stérilisation à la vapeur selon l'une quelconque des revendications 1 à 8, où le générateur de vapeur comprend un corps de four (101) et un dispositif de chauffage électrique (104), une cavité de réception (1011) pour recevoir de l'eau est définie dans le corps de four de celui-ci, la cavité de réception a une entrée d'eau et la sortie de vapeur, le dispositif de chauffage électrique est adapté pour être relié à une source d'énergie par un cordon d'alimentation (95) et le réfrigérateur de stérilisation à la vapeur comprend également :
une pompe de transport ayant une sortie d'eau en communication avec l'entrée d'eau du générateur de vapeur ;
un dispositif de protection de commande de température disposé sur une surface externe du corps de four et configuré pour détecter une température de la surface externe du corps de four, le dispositif de protection de commande de température étant relié au cordon d'alimentation et s'éteignant automatiquement lorsque la température de la surface externe du corps de four est supérieure ou égale à une température prédéfinie ;
un capteur de température disposé sur la surface externe du corps de four et configuré pour détecter la température de la surface externe du corps de four ; et
un dispositif de commande relié au capteur de température et au dispositif de chauffage électrique et commandant le dispositif de chauffage électrique pour qu'il arrête de chauffer lorsque la température de la surface externe du corps de four est supérieure ou égale à la température prédéfinie.

10. Réfrigérateur de stérilisation à la vapeur selon la revendication 9, où le dispositif de commande (83) est relié à un moteur électrique de la pompe de transport (53) de la pompe de transport pour surveiller un courant de fonctionnement du moteur électrique de la pompe de transport, le réfrigérateur de stérilisation à la vapeur comprend en outre un premier voyant lumineux (841) et un deuxième voyant lumineux (842), chacun du premier voyant lumineux et du deuxième voyant lumineux étant relié au dispositif de commande, où, lorsque le courant de fonctionnement du moteur électrique de la pompe de transport se trouve au sein d'une plage prédéfinie et que le dispositif de chauffage électrique arrête de chauffer, le dispositif de commande commande le premier voyant lumineux pour qu'il brille ou scintille, et lorsque le courant de fonctionnement du moteur électrique de la pompe de transport dépasse la plage prédéfinie et que le dispositif de chauffage électrique arrête de chauffer, le dispositif de commande commande le deuxième voyant lumineux pour qu'il brille ou scintille ; ou
comprend en outre un moyen de rappel vocal (86) relié au dispositif de commande, où, lorsqu'un courant de fonctionnement d'un moteur électrique de la pompe de transport se trouve au sein d'une plage prédéfinie et que le dispositif de chauffage électrique arrête de chauffer, le dispositif de commande commande le moyen de rappel vocal pour qu'il émette une première voix de rappel, et lorsque le courant de fonctionnement du moteur électrique de la pompe de transport dépasse la plage prédéfinie et que le dispositif de chauffage électrique arrête de chauffer, le dispositif de commande commande le rappel vocal pour qu'il émette une deuxième voix de rappel.

11. Réfrigérateur de stérilisation à la vapeur selon l'une quelconque des revendications 1 à 10, comprenant en outre :
une jauge de niveau d'eau (87) configurée pour détecter un niveau d'eau dans un réservoir d'eau (50) ;
un voyant lumineux de manque d'eau (88) et un voyant lumineux de pleine eau (89) tous deux disposés dans le compartiment de réfrigération (21) ; et
un dispositif de commande (83) relié à chacun de la jauge de niveau d'eau, du voyant lumineux de manque d'eau, du voyant lumineux de pleine eau et du générateur de vapeur, où le dispositif de commande (83) commande le voyant lumineux de manque d'eau pour qu'il brille ou scintille lorsqu'une valeur de détection de niveau d'eau de la jauge de niveau d'eau est plus petite qu'une première valeur prédéfinie, et commande le voyant lumineux de pleine eau pour qu'il brille ou scintille lorsque la valeur de détection du niveau d'eau de la jauge de niveau d'eau est supérieure à une deuxième valeur prédéfinie.

12. Procédé de commande pour un réfrigérateur de stérilisation à la vapeur (1) selon l'une quelconque des revendications précédentes, où le réfrigérateur de stérilisation à la vapeur comprend un corps de réfrigérateur (20) définissant un compartiment de réfrigération (21) dans celui-ci, un compresseur (22) et un générateur de vapeur (10), et le procédé de commande comprend les étapes :
de commande du générateur de vapeur (10) pour qu'il fonctionne de manière à fournir de la vapeur au compartiment de réfrigération (21) ;
de détection d'une température dans le compartiment de réfrigération (21), et de démarrage du chronométrage lorsqu'une valeur de température détectée atteint une première valeur prédéfinie ;
de commande du générateur de vapeur (10) pour qu'il s'arrête lorsque le chronométrage atteint une première durée prédéfinie ; et
de détection de la température dans le compartiment de réfrigération (21), et de commande du générateur de vapeur (10) pour qu'il s'arrête lorsque la valeur de température détectée atteint une deuxième valeur prédéfinie.

13. Procédé de commande selon la revendication 12, où la première valeur prédéfinie est de 60 à 80 degrés Celsius, et la première durée prédéfinie est de 10 à 40 minutes ; ou
comprenant en outre : la comparaison de la valeur de température détectée avec la première valeur prédéfinie avant que le chronométrage n'atteigne la première durée prédéfinie, la commande du générateur de vapeur (10) pour qu'il s'arrête après un retard d'une deuxième durée prédéfinie lorsque la valeur de température détectée est supérieure à la première valeur prédéfinie, et la commande du générateur de vapeur (10) pour qu'il fonctionne lorsque la valeur de température détectée est plus petite que la première valeur prédéfinie ou égale à celle-ci ; ou
où de l'eau est amenée au générateur de vapeur (10) lorsqu'une température du générateur de vapeur (10) atteint 50 degrés Celsius à 80 degrés Celsius.

14. Procédé de commande selon la revendication 12,
où la première valeur prédéfinie est de 60 à 80 degrés Celsius, la deuxième valeur prédéfinie est de 160 à 200 degrés Celsius, et la première durée prédéfinie est de 10 à 40 minutes.

15. Procédé de commande selon l'une quelconque des revendications 12 à 14, où, après que le générateur de vapeur (10) est commandé pour amener de la vapeur au compartiment de réfrigération (21) pendant une troisième durée prédéfinie, et lorsque la température dans le compartiment de réfrigération est plus petite que la première valeur prédéfinie, le générateur de vapeur est commandé pour qu'il s'arrête ; et facultativement
où la troisième durée prédéfinie est de 35 à 45 minutes.
